# EUROPEAN PATENT SPECIFICATION

(11) **EP 1 194 164 B1**
(45) Date of publication and mention of the grant of the patent: **20.12.2006**
(21) Application number: 00941708.0
(22) Date of filing: 23.06.2000
(51) Int. Cl.: A61K 39/00, A61K 39/40, A61K 39/395, A61K 38/00, A61K 38/06, G01N 33/53, C12N 5/06, C07K 16/00, C07K 16/28, A61K 49/00

(54) **PRION PROTEIN PEPTIDES AND USES THEREOF**
PRION PROTEIN PEPTIDE UND DEREN VERWENDUNG
PEPTIDES PROTEINIQUES DU PRION ET UTILISATIONS ASSOCIEES

(30) Priority: 23.06.1999 US 140634 P
(43) Date of publication of application: 10.04.2002
(73) Proprietor: Caprion Pharmaceuticals, Inc., Montreal, Quebec H4S 2C8 (CA)
(72) Inventor: CASHMAN, Neil, R., Toronto, Ontario M3B 1M5 (CA); PARAMITHIOTIS, Eustache, Boucherville, Quebec J4B 1B9 (CA); SLON-USAKIEWICZ, Jacek, Pointe-Claire, Quebec H9R 3T4 (CA); HAGHIGHAT, Ashkan, Montreal, Quebec H3W 2C6 (CA); PINARD, Marc, Montreal, Quebec H3G 1X6 (CA); LAWTON, Trebor, Gorham, Maine 04038 (US)
(74) Representative: Cornish, Kristina Victoria Joy
(86) International application number: PCT/US2000/017455
(87) International publication number: WO 2000/078344

(56) References cited:
- EP-A1- 0 861 900
- WO-A1-93/11155
- WO-A1-93/23432
- WO-A1-99/19360
- US-A- 5 750 361
- US-A- 5 773 572
- US-A- 5 846 533
- US-A- 5 891 641
- VORBERG I ET AL: "A Novel Epitope for the Specific Detection of Exogenous Prion Proteins in Transgenic Mice and Transfected Murine Cell Lines" VIROLOGY, ACADEMIC PRESS,ORLANDO, US, vol. 255, no. 1, 1 March 1999 (1999-03-01), pages 26-31, XP004467103 ISSN: 0042-6822
- KORTH C ET AL: "PRION (PRPSC)-SPECIFIC EPITOPE DEFINED BY A MONOCLONAL ANTIBODY" NATURE, MACMILLAN JOURNALS LTD. LONDON, GB, vol. 390, no. 6655, 6 November 1997 (1997-11-06), pages 74-77, XP002069611 ISSN: 0028-0836
- CASHMAN N R ET AL: "A PRION-SPECIFIC IMMUNOLOGICAL EPITOPE" ABSTRACTS OF THE SOCIETY FOR NEUROSCIENCE, SOCIETY FOR NEUROSCIENCE, WASHINGTON, DC, US, vol. 27, no. 2, 2001, page 1743, XP001176739 ISSN: 0190-5295

### SEQUENCE LISTING

<110> Caprion Pharmeceuticals, Inc.
<120> PRION PROTEIN PEPTIDES AND USES THEREOF
<130> 50111/002WO2
<150> 60/140,634
   <151> 1999-06-23
<160> 34
<170> Fast SEQ for Windows Version 4.0
<210> 1
   <211> 4
   <212> PRT
   <213> Artificial Sequence
<220>
   <223> Synthetic peptide
<221> VARIANT
   <222> (1)...(4)
   <223> Xaa = Any Amino Acid
<400> 1
   Xaa Tyr Tyr Xaa
    1
<210> 2
   <211> 7
   <212> PRT
   <213> Artificial Sequence
<220>
   <223> Synthetic peptide
<221> VARIANT
   <222> (1)...(7)
   <223> Xaa = Any Amino Acid
<400> 2
   Xaa Tyr Tyr Xaa Tyr Tyr Xaa
    1               5
<210> 3
   <211> 10
   <212> PRT
   <213> Artificial Sequence
<220>
   <223> Synthetic peptide
<221> VARIANT
<222> (1)...(10)
   <223> Xaa = Any Amino Acid
<400> 3
   Xaa Tyr Tyr Xaa Tyr Tyr Xaa Tyr Tyr Xaa
    1               5                  10
<210> 4
   <211> 13
   <212> PRT
   <213> Artificial Sequence
<220>
   <223> Synthetic peptide
<221> VARIANT
   <222> (1)...(13)
   <223> Xaa = Any Amino Acid
<400> 4
   Xaa Tyr Tyr Xaa Tyr Tyr Xaa Tyr Tyr Xaa Tyr Tyr Xaa
    1               5                  10
<210> 5
   <211> 16
   <212> PRT
   <213> Artificial Sequence
<220>
   <223> Synthetic peptide
<221> VARIANT
   <222> (1) ... (16)
   <223> Xaa = Any Amino Acid
<400> 5
   Xaa Tyr Tyr Xaa Tyr Tyr Xaa Tyr Tyr Xaa Tyr Tyr Xaa Tyr Tyr Xaa
    1               5                  10                  15
<210> 6
   <211> 19
   <212> PRT
   <213> Artificial Sequence
<220>
   <223> Synthetic peptide
<221> VARIANT
   <222> (1)...(19)
   <223> Xaa = Any Amino Acid
<400> 6
   Xaa Tyr Tyr Xaa Tyr Tyr Xaa Tyr Tyr Xaa Tyr Tyr Xaa Tyr Tyr Xaa
    1               5                  10                  15
   Tyr Tyr Xaa
<210> 7
<211> 22
   <212> PRT
   <213> Artificial Sequence
<220>
   <223> Synthetic peptide
<221> VARIANT
   <222> (1)...(22)
   <223> Xaa = Any Amino Acid
<400> 7
   Xaa Tyr Tyr Xaa Tyr Tyr Xaa Tyr Tyr Xaa Tyr Tyr Xaa Tyr Tyr Xaa
    1               5                  10                  15
   Tyr Tyr Xaa Tyr Tyr Xaa
               20
<210> 8
   <211> 25
   <212> PRT
   <213> Artificial Sequence
<220>
   <223> Synthetic peptide
   <221> VARIANT
   <222> (1) ... (25)
   <223> Xaa = Any Amino Acid
<400> 8
   Xaa Tyr Tyr Xaa Tyr Tyr Xaa Tyr Tyr Xaa Tyr Tyr Xaa Tyr Tyr Xaa
    1               5                  10                  15
   Tyr Tyr Xaa Tyr Tyr Xaa Tyr Tyr Xaa
               20                  25
<210> 9
   <211> 28
   <212> PRT
   <213> Artificial Sequence
<220>
   <223> Synthetic peptide
   <221> VARIANT
   <222> (1) ... (28)
   <223> Xaa = Any Amino Acid
<400> 9
   Xaa Tyr Tyr Xaa Tyr Tyr Xaa Tyr Tyr Xaa Tyr Tyr Xaa Tyr Tyr Xaa
    1               5                   10                  15
   Tyr Tyr Xaa Tyr Tyr Xaa Tyr Tyr Xaa Tyr Tyr Xaa
               20                  25
<210> 10
   <211> 31
   <212> PRT
   <213> Artificial Sequence
<220>
   <223> Synthetic peptide
<221> VARIANT
   <222> (1)...(31)
   <223> Xaa = Any Amino Acid
<400> 10
   Xaa Tyr Tyr Xaa Tyr Tyr Xaa Tyr Tyr Xaa Tyr Tyr Xaa Tyr Tyr Xaa
    1               5                   10                  15
   Tyr Tyr Xaa Tyr Tyr Xaa Tyr Tyr Xaa Tyr Tyr Xaa Tyr Tyr Xaa
               20                  25                  30
<210> 11
   <211> 34
   <212> PRT
   <213> Artificial Sequence
<220>
   <223> Synthetic peptide
<221> VARIANT
   <222> (1)...(34)
   <223> Xaa = Any Amino Acid
<400> 11
   Xaa Tyr Tyr Xaa Tyr Tyr Xaa Tyr Tyr Xaa Tyr Tyr Xaa Tyr Tyr Xaa
    1               5                   10                  15
   Tyr Tyr Xaa Tyr Tyr Xaa Tyr Tyr Xaa Tyr Tyr Xaa Tyr Tyr Xaa Tyr
               20                  25                  30
   Tyr Xaa
<210> 12
   <211> 4
   <212> PRT
   <213> Artificial Sequence
<220>
   <223> Synthetic peptide
<221> VARIANT
   <222> (1) ... (4)
   <223> Xaa = Any Amino Acid
<400> 12
   Xaa Tyr Tyr Arg
    1
<210> 13
   <211> 4
   <212> PRT
   <213> Artificial Sequence
<220>
   <223> Synthetic peptide
<221> VARIANT
   <222> (1)...(4)
   <223> Xaa = Any Amino Acid
<400> 13
   Xaa Tyr Tyr Gln
    1
<210> 14
   <211> 4
   <212> PRT
   <213> Artificial Sequence
<220>
   <223> Synthetic peptide
<221> VARIANT
   <222> (1)...(4)
   <223> Xaa = Any Amino Acid
<400> 14
   Xaa Tyr Tyr Asp
    1
<210> 15
   <211> 13
   <212> PRT
   <213> Artificial Sequence
<220>
   <223> Synthetic peptide
<221> VARIANT
   <222> (1)...(13)
   <223> Xaa = Any Amino Acid
<400> 15
   Xaa Tyr Tyr Xaa Xaa Tyr Tyr Xaa Tyr Tyr Tyr Tyr Xaa
   1 5 10
<210> 16
   <211> 16
   <212> PRT
   <213> Artificial Sequence
<220>
   <223> Synthetic peptide
<221> VARIANT
   <222> (1) ... (16)
   <223> Xaa = Any Amino Acid
<400> 16
   Xaa Tyr Tyr Xaa Xaa Tyr Tyr Xaa Tyr Tyr Tyr Tyr Xaa Tyr Tyr Xaa
   1 5 10 15
<210> 17
   <211> 19
   <212> PRT
   <213> Artificial Sequence
<220>
   <223> Synthetic peptide
<221> VARIANT
   <222> (1) ... (19)
   <223> Xaa = Any Amino Acid
<400> 17
   Xaa Tyr Tyr Xaa Xaa Tyr Tyr Xaa Tyr Tyr Tyr Tyr Xaa Tyr Tyr Xaa
   1 5 10 15
   Tyr Tyr Xaa
<210> 18
   <211> 22
   <212> PRT
   <213> Artificial Sequence
<220>
   <223> Synthetic peptide
<221> VARIANT
   <222> (1)...(22)
   <223> Xaa = Any Amino Acid
<400> 18
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa
               20
<210> 19
   <211> 25
   <212> PRT
   <213> Artificial Sequence
<220>
   <223> Synthetic peptide
<221> VARIANT
   <222> (1)...(25)
   <223> Xaa = Any Amino Acid
<400> 19
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa
               20                  25
<210> 20
   <211> 28
   <212> PRT
   <213> Artificial Sequence
<220>
   <223> Synthetic peptide
   <221> VARIANT
   <222> (1)...(28)
   <223> Xaa = Any Amino Acid
<400> 20
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa Tyr Tyr Xaa
               20                  25
<210> 21
   <211> 31
   <212> PRT
   <213> Artificial Sequence
<220>
   <223> Synthetic peptide
   <221> VARIANT
   <222> (1)...(31)
   <223> Xaa = Any Amino Acid
<400> 21
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa Tyr Tyr Xaa Tyr Tyr Xaa
               20                  25                  30
<210> 22
   <211> 34
   <212> PRT
   <213> Artificial Sequence
<220>
   <223> Synthetic peptide
   <221> VARIANT
   <222> (1)...(34)
   <223> Xaa = Any Amino Acid
<400> 22
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa Tyr Tyr Xaa Tyr Tyr Xaa Tyr
               20                  25                  30
   Tyr Xaa
<210> 23
   <211> 37
   <212> PRT
   <213> Artificial Sequence
<220>
   <223> Synthetic peptide
   <221> VARIANT
   <222> (1)...(37)
   <223> Xaa = Any Amino Acid
<400> 23
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa Tyr Tyr Xaa Tyr Tyr Xaa Tyr
               20                  25                  30
   Tyr Xaa Tyr Tyr Xaa
           35
<210> 24
   <211> 40
   <212> PRT
   <213> Artificial Sequence
<220>
   <223> Synthetic peptide
   <221> VARIANT
   <222> (1)...(40)
   <223> Xaa = Any Amino Acid
<400> 24
   Xaa Tyr Tyr Xaa Xaa Tyr Tyr Xaa Tyr Tyr Tyr Tyr Xaa Tyr Tyr Xaa
    1               5                  10                  15
   Tyr Tyr Xaa Tyr Tyr Xaa Tyr Tyr Xaa Tyr Tyr Xaa Tyr Tyr Xaa Tyr
               20                  25                  30
   Tyr Xaa Tyr Tyr Xaa Tyr Tyr Xaa
           35                  40
<210> 25
   <211> 10
   <212> PRT
   <213> Artificial Sequence
<220>
   <223> Synthetic peptide
   <221> VARIANT
   <222> (1)...(10)
   <223> Xaa = Any Amino Acid
<400> 25
   Xaa Tyr Tyr Arg Arg Tyr Tyr Arg Tyr Tyr
    1               5                  10
<210> 26
   <211> 264
   <212 PRT
   <213> Bos taurus
<400> 26
   Met Val Lys Ser His Ile Gly Ser Trp Ile Leu Val Leu Phe Val Ala
    1               5                  10                  15
   Met Trp Ser Asp Val Gly Leu Cys Lys Lys Arg Pro Lys Pro Gly Gly
               20                  25                  30
   Gly Trp Asn Thr Gly Gly Ser Arg Tyr Pro Gly Gln Gly Ser Pro Gly
           35                  40                  45
   Gly Asn Arg Tyr Pro Pro Gln Gly Gly Gly Gly Trp Gly Gln Pro His
       50                  55                  60
   Gly Gly Gly Trp Gly Gln Pro His Gly Gly Gly Trp Gly Gln Pro His
   65                  70                  75                  80
   Gly Gly Gly Trp Gly Gln Pro His Gly Gly Gly Trp Gly Gln Pro His
                   85                  90                  95
   Gly Gly Gly Gly Trp Gly Gln Gly Gly Thr His Gly Gln Trp Asn Lys
               100                 105                 110
   Pro Ser Lys Pro Lys Thr Asn Met Lys His Val Ala Gly Ala Ala Ala
           115                 120                 125
   Ala Gly Ala Val Val Gly Gly Leu Gly Gly Tyr Met Leu Gly Ser Ala
       130                 135                 140
   Met Ser Arg Pro Leu Ile His Phe Gly Ser Asp Tyr Glu Asp Arg Tyr
   145                 150                 155                 160
   Tyr Arg Glu Asn Met His Arg Tyr Pro Asn Gln Val Tyr Tyr Arg Pro
                   165                 170                 175
   Val Asp Gln Tyr Ser Asn Gln Asn Asn Phe Val His Asp Cys Val Asn
               180                 185                 190
   Ile Thr Val Lys Glu His Thr Val Thr Thr Thr Thr Lys Gly Glu Asn
           195                 200                 205
   Phe Thr Glu Thr Asp Ile Lys Met Met Glu Arg Val Val Glu Gln Met
       210                 215                 220
   Cys Ile Thr Gln Tyr Gln Arg Glu Ser Gln Ala Tyr Tyr Gln Arg Gly
   225                 230                 235                 240
   Ala Ser Val Ile Leu Phe Ser Ser Pro Pro Val Ile Leu Leu Ile Ser
                   245                 250                 255
   Phe Leu Ile Phe Leu Ile Val Gly
               260
<210> 27
   <211> 253
   <212> PRT
   <213> Homo sapiens
<400> 27
<210> 28
   <211> 256
   <212> PRT
   <213> Ovis aries
<400> 28
   Met Val Lys Ser His Ile Gly Ser Trp Ile Leu Val Leu Phe Val Ala
    1               5                  10                  15
   Met Trp Ser Asp Val Gly Leu Cys Lys Lys Arg Pro Lys Pro Gly Gly
               20                  25                  30
   Gly Trp Asn Thr Gly Gly Ser Arg Tyr Pro Gly Gln Gly Ser Pro Gly
           35                  40                  45
   Gly Asn Arg Tyr Pro Pro Gln Gly Gly Gly Gly Trp Gly Gln Pro His
       50                  55                  60
   Gly Gly Gly Trp Gly Gln Pro His Gly Gly Gly Trp Gly Gln Pro His
   65                  70                  75                  80
   Gly Gly Gly Trp Gly Gln Pro His Gly Gly Gly Gly Trp Gly Gln Gly
                   85                  90                  95
   Gly Ser His Ser Gln Trp Asn Lys Pro Ser Lys Pro Lys Thr Asn Met
               100                 105                 110
   Lys His Val Ala Gly Ala Ala Ala Ala Gly Ala Val Val Gly Gly Leu
           115                 120                 125
   Gly Gly Tyr Met Leu Gly Ser Ala Met Ser Arg Pro Leu Ile His Phe
       130                 135                 140
   Gly Asn Asp Tyr Glu Asp Arg Tyr Tyr Arg Glu Asn Met Tyr Arg Tyr
   145                 150                 155                 160
   Pro Asn Gln Val Tyr Tyr Arg Pro Val Asp Arg Tyr Ser Asn Gln Asn
                   165                 170                 175
   Asn Phe Val His Asp Cys Val Asn Ile Thr Val Lys Gln His Thr Val
               180                 185                 190
   Thr Thr Thr Thr Lys Gly Glu Asn Phe Thr Glu Thr Asp Ile Lys Ile
           195                 200                 205
   Met Glu Arg Val Val Glu Gln Met Cys Ile Thr Gln Tyr Gln Arg Glu
       210                 215                 220
   Ser Gln Ala Tyr Tyr Gln Arg Gly Ala Ser Val Ile Leu Phe Ser Ser
   225                 230                 235                 240
   Pro Pro Val Ile Leu Leu Ile Ser Phe Leu Ile Phe Leu Ile Val Gly
                   245                 250                 255
<210> 29
   <211> 254
   <212> PRT
   <213> Mus musculus
<400> 29
   Met Ala Asn Leu Gly Tyr Trp Leu Leu Ala Leu Phe Val Thr Met Trp
    1               5                  10                  15
   Thr Asp Val Gly Leu Cys Lys Lys Arg Pro Lys Pro Gly Gly Trp Asn
               20                  25                  30
   Thr Gly Gly Ser Arg Tyr Pro Gly Gln Gly Ser Pro Gly Gly Asn Arg
           35                  40                  45
   Tyr Pro Pro Gln Gly Gly Thr Trp Gly Gln Pro His Gly Gly Gly Trp
       50                  55                  60
   Gly Gln Pro His Gly Gly Ser Trp Gly Gln Pro His Gly Gly Ser Trp
   65                  70                  75                  80
   Gly Gln Pro His Gly Gly Gly Trp Gly Gln Gly Gly Gly Thr His Asn
                   85                  90                  95
   Gln Trp Asn Lys Pro Ser Lys Pro Lys Thr Asn Leu Lys His Val Ala
               100                 105                 110
   Gly Ala Ala Ala Ala Gly Ala Val Val Gly Gly Leu Gly Gly Tyr Met
           115                 120                 125
   Leu Gly Ser Ala Met Ser Arg Pro Met Ile His Phe Gly Asn Asp Trp
       130                 135                 140
   Glu Asp Arg Tyr Tyr Arg Glu Asn Met Tyr Arg Tyr Pro Asn Gln Val
   145                 150                 155                 160
   Tyr Tyr Arg Pro Val Asp Gln Tyr Ser Asn Gln Asn Asn Phe Val His
                   165                 170                 175
   Asp Cys Val Asn Ile Thr Ile Lys Gln His Thr Val Thr Thr Thr Thr
               180                 185                 190
   Lys Gly Glu Asn Phe Thr Glu Thr Asp Val Lys Met Met Glu Arg Val
           195                 200                 205
   Val Glu Gln Met Cys Val Thr Gln Tyr Gln Lys Glu Ser Gln Ala Tyr
       210                 215                 220
   Tyr Asp Gly Arg Arg Ser Ser Ser Thr Val Leu Phe Ser Ser Pro Pro
   225                 230                 235                 240
   Val Ile Leu Leu Ile Ser Phe Leu Ile Phe Leu Ile Val Gly
                   245                 250
<210> 30
   <211> 254
   <212> PRT
   <213> Mesocricetus auratus
<400> 30
   Met Ala Asn Leu Ser Tyr Trp Leu Leu Ala Leu Phe Val Ala Met Trp
    1               5                  10                  15
   Thr Asp Val Gly Leu Cys Lys Lys Arg Pro Lys Pro Gly Gly Trp Asn
               20                  25                  30
   Thr Gly Gly Ser Arg Tyr Pro Gly Gln Gly Ser Pro Gly Gly Asn Arg
           35                  40                  45
   Tyr Pro Pro Gln Gly Gly Gly Thr Trp Gly Gln Pro His Gly Gly Gly
       50                  55                  60
   Trp Gly Gln Pro His Gly Gly Gly Trp Gly Gln Pro His Gly Gly Gly
   65                  70                  75                  80
   Trp Gly Gln Pro His Gly Gly Gly Trp Gly Gln Gly Gly Gly Thr His
                   85                  90                  95
   Asn Gln Trp Asn Lys Pro Ser Lys Pro Lys Thr Asn Met Lys His Met
               100                 105                 110
   Ala Gly Ala Ala Ala Ala Gly Ala Val Val Gly Gly Leu Gly Gly Tyr
           115                 120                 125
   Met Leu Gly Ser Ala Met Ser Arg Pro Met Met His Phe Gly Asn Asp
       130                 135                 140
   Trp Glu Asp Arg Tyr Tyr Arg Glu Asn Met Asn Arg Tyr Pro Asn Gln
   145                 150                 155                 160
   Val Tyr Tyr Arg Pro Val Asp Gln Tyr Asn Asn Gln Asn Asn Phe Val
                   165                 170                 175
   His Asp Cys Val Asn Ile Thr Ile Lys Gln His Thr Val Thr Thr Thr
               180                 185                 190
   Thr Lys Gly Glu Asn Phe Thr Glu Thr Asp Ile Lys Ile Met Glu Arg
           195                 200                 205
   Val Val Glu Gln Met Cys Thr Thr Gln Tyr Gln Lys Glu Ser Gln Ala
       210                 215                 220
   Tyr Tyr Asp Gly Arg Arg Ser Ser Ala Val Leu Phe Ser Ser Pro Pro
   225                 230                 235                 240
   Val Ile Leu Leu Ile Ser Phe Leu Ile Phe Leu Met Val Gly
                   245                 250
<210> 31
   <211> 9
   <212> PRT
   <213> Artificial Sequence
<220>
   <223> Synthetic peptide
<400> 31
   Tyr Tyr Arg Arg Tyr Tyr Arg Tyr Tyr
    1               5
<210> 32
   <211> 4
   <212> PRT
   <213> Artificial Sequence
<220>
   <223> Synthetic peptide
<400> 32
   Cys Tyr Tyr Arg
   1
<210> 33
   <211> 10
   <212> PRT
   <213> Artificial Sequence
<220>
   <223> Synthetic peptide
<400> 33
   Cys Tyr Tyr Arg Arg Tyr Tyr Arg Tyr Tyr
   1 5 10
<210> 34
   <211> 10
   <212> PRT
   <213> Artificial Sequence
<220>
   <223> Synthetic peptide
<400> 34
   Cys Lys Tyr Glu Asp Arg Tyr Tyr Arg Glu
   1 5 10

## Description

### Background of the Invention

This invention relates to PrP^{Sc}-specific antibodies and to peptides used for their generation. These antibodies are suitable for detecting PrP^{Sc} in a sample, and for purifying PrP^{Sc}. Additionally, the invention relates to diagnostic aids for the detection of PrP^{Sc}, pharmaceuticals that contain or mimic PrP^{Sc}-specific conformational epitopes, and methods for prion decontamination.

Prions are infectious agents that are associated with neurodegenerative syndromes characterized by spongiform change (e.g., microcavitation of the brain, usually predominant in gray matter), neuronal cell loss, astrocytic proliferation disproportionate to neuronal loss, and accumulation of an abnormal amyloidogenic protein, sometimes in discrete plaques in the brain. It is possible that neurodegeneration in prion diseases shares certain underlying mechanisms with other more common neurodegenerative syndromes such as Alzheimer's Disease, amyotrophic lateral sclerosis, and Parkinson's disease.

The agents that transmit these diseases differ markedly from viruses and viroids in that no chemical or physical evidence for a nucleic acid component has been reproducibly detected in infectious materials (Prusiner, *Science,* 216: 136-144, 1982). A major step in the study of prions and the diseases that they cause was the discovery and purification of a protein designated prion protein (PrP) (Bolton et al., *Science* 218:1309-11, 1982; Prusiner et al., *Biochemistry,* 21:6942-50, 1982; McKinley et al., *Cell,* 35:57-62, 1983). When purified using proteinase K digestion, a 27-30 kD protease-resistant protein was discovered in scrapie-affected hamster brain, and was termed PrP 27-30, later found to be a fragment of PrP^{Sc} (Bolton, *Science,* 218:1309-1311, 1982).

According to the prion hypothesis, prion infectivity resides in PrP^{Sc}. PrP^{Sc} is at least strongly associated with infectivity and appears to be a reliable surrogate for prion infection. PrP^{Sc} is a conformational variant of a host-encoded cellular protein designated PrP^{C} (Oesch et al., *Cell,* 40:735-746, 1985), which is a glycosylphosphatidylinositol (GPI)-linked cell surface protein with a molecular mass of 33-35 kD.

PrP^{C} has been isolated from normal brain, and has been found to be protease-sensitive and not associated with scrapie disease-producing activity (Bendheim et al., *Ciba Found. Symp.* 164-177, 988). According to the prion theory, PrP^{C} converts into PrP^{Sc} autocatalytically (Prusiner, *Proc. Natl. Acad. Sci, USA* 95:13363-83, 1998). More recently, it was reported that PrP^{C} can be converted to a protease-resistant form *in vitro* by PrP^{Sc} (Kocisko et al., *Nature,* 370:471-473, 1994). PrP^{C} is an evolutionarily conserved membrane protein for which the actual biological or physiological function is unknown. Mice devoid of PrP^{C} are viable and show no obvious signs of neurological and physical impairment (Bueler et al., *Nature,* 356:577-582, 1992). Additionally, these mice are not susceptible to prion infection, underscoring the central importance of PrP in the replication of infectivity (Bueler et al., *Cell,* 73:1339-1347, 1993: Prusiner et al., *Proc. Natl. Acad. Sci. USA,* 90:10608-10612, 1993). Targeted investigations of PrP knockout mice revealed impaired synaptic function (Collinge et al., *Nature,* 370:295-297, 1994) and altered sleep regulation (Tobler et al., *J Neurosci.,* 17:1869-79). Moreover, PrP^{C} has been shown to modify T cell activation induced by concanavalin A stimulation (Cashman et al., *Cell* 61:185-192, 1990), indicating a functional role for the protein.

The prion diseases are a group of rapidly progressive, fatal, and untreatable neurodegenerative syndromes. Human prion diseases include Creutzfeldt-Jakob disease (CJD), which has sporadic, iatrogenic, and familial forms; and variant CJD ("vCJD"), likely derived from the consumption of cattle tissues contaminated with the agent of bovine spongiform encephalopathy (reviewed in Cashman, *Can. Med. Assoc. J.* 157:1381-5, 1997). CJD has been accidentally transmitted between humans by contaminated cadaveric pituitary hormones, dura mater transplantation, neurosurgical instrumentation, and corneal transplantation (Brown et al., *Lancet* 340:24-7, 1992). The potential risk of transmitting CJD through blood and blood products is of worldwide concern. Moreover, scrapie in sheep and goats is a common and economically important prion-related disease in North America, as is bovine spongiform encephalopathy (BSE) in Great Britain. According to Britain's Ministry of Agriculture, Fisheries and Food, more than 4,347,380 cattle have been destroyed, because they were deemed old enough to conceivably harbor the disease agent. The Ministry of Agriculture has estimated that the total cost of the epidemic will reach $7.13 billion by 2002. More than 173,000 bovines from all over Britain have been confirmed to be infected, and hundreds of thousands more might have entered the food supply undetected.

Additionally, the United States and Canada have now implemented a blood donor deferral for individuals who resided in the UK during the early and peak years of the BSE epidemic. Such a restriction has been adopted as a precaution against the risk of transmitting a vCJD, which to date has afflicted over 60 Britons since 1996. The Canadian Blood Services estimates that 120,000 of its 600,000 active donors, or 22%, have visited Britain since 1980 (Montreal Gazette, May 6,1999). Many new donors have been recruited to replace the loss, raising several concerns. One such concern is that the blood of new donors is not as safe since it has only been screened once for illnesses such as hepatitis and human immunodeficiency virus.

Accordingly, a need exists for diagnostic methods suitable for mass screening of prion infected blood or tissues. The availability of antibodies that distinguish PrP^{C} from PrP^{Sc} would therefore be of great value in development of a test for prion infection. Furthermore, a need exists for therapeutic agents that prevent and/or treat prion diseases.

### Summary of the Invention

In a first aspect, the invention provides an antibody or fragment thereof that binds with high binding affinity to a YYX epitope of a mammalian PrP^{Sc}.

In a second aspect, the invention provides a hybridoma cell line that produces a monoclonal antibody that binds with high binding affinity to a YYX epitope of a mammalian PrP^{Sc}.

In a third aspect, the invention provides a composition comprising the antibody of the first aspect.

In a fourth aspect, the invention provides an immunological test kit comprising the antibody of the first aspect and a means for detecting the antibody. In a fifth aspect, the invention provides a method for detecting PrP^{Sc} in a biological sample, said method comprising the steps of:
(a) contacting said biological sample with an antibody of the first aspect under conditions that allow for complex formation between said antibody and PrP^{Sc}; and
(b) detecting said complexes as an indication that PrP^{Sc} is present in said biological sample.

In a sixth aspect, the invention provides a method for generating an antibody that binds with high binding affinity to a mammalian PrP^{Sc}, said method comprising the steps of:
(a) providing a prion protein peptide comprising an accessible epitope having two or more amino acid side chains;
(b) immunising a non-human mammal with said prion protein peptide of step (a); and
(c) purifying an antibody that binds with high binding affinity to a YYX epitope of a mammalian PrP^{Sc} from a tissue of said mammal or from a hybridoma made using said tissue.

In a seventh aspect, the invention provides a method for decontaminating PrP^{Sc} from a biological sample, said method comprising the steps of:
(a) treating the biological sample with an antibody of the first aspect for a period of time sufficient to permit the formation of an anti-PrP^{Sc} antibody:PrP^{Sc} complex; and
(b) recovering said anti-PrP^{Sc} antibody:PrP^{Sc} complex from said biological sample.

In an eighth aspect, the invention provides a method of inhibiting PrP^{Sc} in a biological sample isolated from the human or animal body, said method comprising:
treating the biological sample with an antibody of the first aspect for a period of time sufficient to permit the formation of an anti-PrP^{Sc} antibody:PrP^{Sc} complex.

In a ninth aspect, the invention provides the use of an antibody of the first aspect in the manufacture of a medicament for treating or preventing prion diseases.

Particular embodiments of each of the aspects of the invention are set out in the dependent claims.

As is discussed herein, evidence is provided demonstrating that a YYX continuous epitope of PrP is useful for generating antibodies specific for PrP^{Sc}. In particular, we have demonstrated that immunization protocols utilizing a short continuous synthetic peptide from the PrP sequence resulted in the generation of high-affinity polyclonal and monoclonal antibodies specific to PrP^{Sc}. Moreover, such antibodies were also observed to lack detectable reactivity with PrP^{C}. In one example, a peptide including a YYR sequence was chosen based on molecular modeling analysis of the conformational change from PrP^{C} to PrP^{Sc}, that predicted a sequence which is solvent-accessible on the molecular surface of the PrP^{Sc} isoform of the protein.

Accordingly, the invention features epitope-specific anti-PrP antibodies or fragments thereof that bind with high binding affinity to a continuous YYX epitope of a mammalian PrP^{Sc}. Preferably, the antibody binds to a YYR epitope; a YYQ epitope; or a YYD epitope of a mammalian PrP^{Sc}. In preferred embodiments, the antibody is a monoclonal or a polyclonal antibody. Such antibodies include IgG, IgM, IgE, IgD, or IgA antibodies, as well as fragments such as Fab or Fv fragments. Such anti-PrP antibodies are advantageously directed against a particular PrP^{Sc} epitope. In addition, antibodies that bind to PrP can be used to quantitate PrP^{Sc} in any standard diagnostic assay.

In the fifth aspect, the invention features the use of epitope-specific anti-PrP antibodies in an immunological detection procedure for the diagnosis of infective disease-specific prions. Anti-PrP antibodies that react specifically with PrP^{Sc} can be prepared using an appropriately adapted PrP peptide as has been illustrated herein. The invention particularly relates to diagnostic aids that contain the PrP peptide, and/or epitope-specific anti-PrP antibodies. In addition, the invention relates to antibodies that selectively bind to disease-specific prion protein and not normal prion protein.

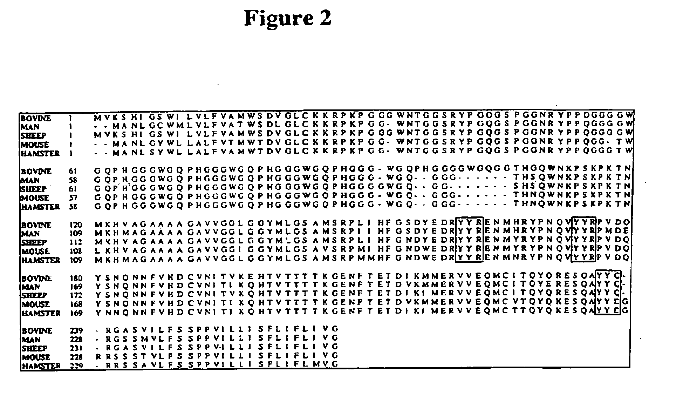

The invention features a prion protein peptide with the sequence tyrosine-tyrosine-arginine (YYR). Preferably, the peptide is a tripeptide that is linked to a carrier, making the peptide more immunogenic, allowing for the preparation of high-affinity anti-PrP antibodies. The synthesis of such a tripeptide is described herein. According to the invention, such short peptides (e. g., the YYR, YYQ, or YYD tripeptides), represent determinants that are accessible in the PrP^{Sc} isoform of the prion protein, but not in the normal PrP^{C} isoform, and/or are clustered in PrP^{Sc} in a manner which allows antibody detection. For example, the YYR tripeptide is contained within two of the three prion protein epitopes; however, this tripeptide has not been previously identified as the specific basis of PrP^{Sc} immunoreactivity. Moreover, such sequences are highly conserved across a number of species including, but not limited to bovine, man, sheep, mouse, and hamster (Figure 2).

The invention further features a synthetic peptide having the formula:
A-Tyr-Tyr-B- (Tyr-Tyr-B)ₙ (SEQ ID NOS: 1-11)
wherein A is either any amino acid or is absent; B is either any amino acid or is absent; and n is from 0 to 10, inclusive. In preferred embodiments, at least one of A and B is not Tyr. In other preferred embodiments, A or B are chosen from Ala, Cys, Asp, Glu, Phe, Gly, His, Ile, Lys, Leu, Met, Asn, Pro, Gln, Arg, Ser, Thr, Val, or Trp. In other preferred embodiments, the peptide is linked to an immunological carrier. Such peptides include, without limitation, A-Tyr-Tyr-Arg (SEQ ID NO: 12); A-TyTyr-Gln (SEQ IDNO: 13); A-Tyr-Tyr-Asp (SEQ ID NO: 14); or any pharmaceutically acceptable salt thereof.

The invention features a synthetic peptide having the formula:
A-Tyr-Tyr-B-C-Tyr-Tyr-D-Tyr-Tyr-(Tyr-Tyr-B)ₙ (SEQ ID NOS: 15-24)
wherein A is either any amino acid or is absent; B is either any amino acid or is absent; C is either any amino acid or is absent; D is either any amino acid or is absent; and n is 0 to 10, inclusive. In preferred embodiments, at least one of A, B, C, and D is not Tyr. In other preferred embodiments, A, B, C, or D are chosen from Ala, Cys, Asp, Glu, Phe, Gly, His, Ile, Lys, Leu, Met, Asn, Pro, Gln, Arg, Ser, Thr, Val, or Trp. In still other preferred embodiments, A is chosen from Ala, Cys, Asp, Glu, Phe, Gly, His, Ile, Lys, Leu, Met, Asn, Pro, Gln, Arg, Ser, Thr, Val, or Trp, and B, C, and D are chosen from Arg, Gln, Asp, Glu, Phe, or Trp. An exemplary peptide includes, without limitation, A-Tyr-Tyr-Arg-Arg-Tyr-Tyr-Arg-Tyr-Tyr (SEQ ID NO: 25); or a pharmaceutically acceptable salt thereof.. In other embodiments, the peptide is linked to an immunological carrier.

The invention relates to short synthetic prion peptides (e. g., three to ten amino acids or four to twelve amino acids, inclusive) having antigencity as a PrP^{Sc}, including one or more of the following: a threonine tetrarepeat found at T189-193 of mouse PrP or the corresponding amino acid residues of human, sheep, goat, or bovine PrP; the M128, M133, or M153 amino acid residues of mouse PrP or the corresponding amino acid residues of human, sheep, goat, or bovine PrP; the H186 amino acid residue of mouse PrP or the corresponding amino acid residue of human, sheep, goat, or bovine PrP; the Q159, Q167, Q185, or Q216 amino acid residues of mouse PrP or the corresponding amino acid residues of human, sheep, goat, or bovine PrP; the N158 amino acid residue of mouse PrP or the corresponding amino acid residue of human, sheep, goat, or bovine PrP; the M128, M133, or M153 amino acid residues of mouse PrP or the corresponding amino acid residues of human, sheep, goat, or bovine PrP; the L124 or L129 amino acid residues of mouse PrP or the corresponding amino acid residues of human, sheep, goat, or bovine PrP; or the I181 or I183 amino acid residues of mouse PrP or the corresponding amino acid residues of human, sheep, goat, or bovine PrP.

Peptides described herein can be prepared by chemical synthesis according to methods known in the art.

In addition, a PrP peptide can be used for preparing epitope-specific anti-PrP^{Sc} antibodies. In particular, the peptide provides the advantages of a highly pure substance, and is suitable for preparing anti-PrP antibodies which can be used to detect PrP^{Sc} in a sample, for example, in standard immunological assays such as immunoprecipitations, ELISA, and flow cytometry. The PrP peptide (e.g., YYR) can be used to prepare both polyclonal epitope-specific anti-PrP^{Sc} antibodies (antisera) and monoclonal epitope-specific anti-PrP antibodies. These antibodies are prepared according to standard methods known in the art, and are preferably bound to a carrier material for the generation of antibodies.

Moreover, compounds which exploit the PrP^{Sc}-specific exposure of YYX can be rationally designed or obtained from combinatorial libraries which mimic the interaction of YYX with anti-YYX antibodies. These compounds are useful in prion diagnostics or as therapies for prion diseases.

In the third aspect, the invention features a pharmaceutical preparation for the therapy and prevention of prion diseases comprising a polyclonal or monoclonal antibody in a pharmaceutical carrier. Such pharmaceuticals contain epitope-specific anti-PrP antibodies according to the invention.

If desired, the peptides and antibodies of the invention can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid. In addition, any of the peptides or antibodies of the invention may be administered to a mammal, particularly a human, in one of the traditional modes (e.g., orally, parenterally, transdermally, or transmucosally), in a sustained release formulation using a biodegradable biocompatible polymer, or by using micelles, gels, and liposomes.

A method of treating or preventing a prion disease in an animal (for example, a human, a bovine, sheep, pig, goat, dog, or cat) is described. The method may involve administering to the animal a therapeutically effective amount of epitope-specific anti-PrP antibody or PrP peptide that blocks the conversion of PrP^{C} to PrP^{Sc}, inhibits PrP^{Sc}:PrP^{Sc} aggregate formation, or blocks the recruitment of PrP^{C} to PrP^{Sc}. The PrP peptide may also be used to immunize the host against prion disease by stimulating the production of host antibodies specific for PrP^{Sc}.

In the fourth and fifth aspects, the invention features methods and kits for detecting PrP^{Sc} in a biological sample.

In the seventh aspect, the invention features methods for decontaminating PrP^{Sc} from a biological sample. The method involves the steps of: (a) treating the biological sample with the polyclonal or monoclonal antibody (or a fragment or analog thereof), the treatment permitting antibody:PrP^{Sc} complex formation; and (b) recovering the antibody:PrP^{Sc} complex from the biological sample. Such a decontamination procedure may also involve the use of perfusing a biological sample with antibody (or a fragment or analog thereof) for the removal or inactivation of PrP^{Sc}.

A method for identifying a compound for the treatment of a prion disease is described. The method includes the steps of (a) measuring the binding of an anti-YYX antibody to PrP^{Sc} in the presence of a test compound; and (b) measuring the binding of the anti-YYX antibody to PrP^{Sc} in the absence of the test compound; wherein a level of binding of the anti-YYX antibody to PrP^{Sc} in the presence of the test compound that is less than the level of binding of the anti-YYX antibody to PrP^{Sc} in the absence of the test compound is an indication that the test compound is a potential therapeutic compound for the treatment of a prion disease. Preferably, the anti-YYX antibody is an anti-YYR antibody, anti-YYD antibody, or anti-YYQ antibody.

A method for identifying a compound for diagnosing a prion disease is described. The method includes the steps of: (a) measuring the binding of an anti-YYX antibody to PrP^{Sc} in the presence of a test compound; and (b) measuring the binding of the anti-YYX antibody to PrP^{Sc} in the absence of the test compound; wherein a level of binding of the anti-YYX antibody to PrP^{Sc} in the presence of the test compound that is less than the level of binding of the anti-YYX antibody to PrP^{Sc} in the absence of the test compound is an indication that the test compound is a potential compound useful for diagnosing a prion disease.

Therapeutic and diagnostic compounds identified according to any of the aforementioned methods are considered.

By "high binding affinity" is meant binding with an affinity constant of less than 10µM, preferably, less than 1 µM, more preferably, less than 100 nM, and, most preferably, less than 10 nM.

By "prion diseases" is meant a group of prion-mediated, rapidly progressive, fatal, and untreatable brain degenerative disorders including, without limitation, Creutzfeldt-Jakob disease (CJD), variant CJD, iatrogenic CJD, familial CJD, Kuru, Gerstmann-Straussler syndrome, and fatal familial insomnia in humans (Prusiner, Science 252: 1515-1522, 1991), scrapie in sheep and goats, and spongiform encephalopathy in cattle, as well as recently described prion diseases in other ruminants and cats.

By "treatment of prion diseases" is meant the ability to reduce, prevent, stabilize, or retard the onset of any symptom associated with prion diseases, particularly those resulting in spongiform change, neuronal cell loss, astrocytic proliferation, accumulation of PrP^{Sc} protein, dementia, or death.

By "purified antibody" is meant antibody that is at least 60%, by weight, free from the proteins and naturally-occurring organic molecules with which it is naturally associated. Preferably, the preparation is at least75%, more preferably at least 90%, and most preferably at least 99%, by weight, antibody, e.g., YYX-specific antibody (e.g., a YYR-, YYQ-, or YYD-specific antibody). A purified antibody may be obtained, for example, by affinity chromatography using substrate-bound YYX and standard techniques.

By "YYX" is meant a peptide having the sequence Tyrosine-Tyrosine-X, where X is any amino acid. By "YYR" is meant a peptide having the sequence Tyrosine-Tyrosine-Arginine. By "YYQ" is meant a peptide having the sequence "Tyrosine-Tyrosine-Glutamine." By "YYD" is meant a peptide having the sequence "Tyrosine-Tyrosine-Aspartic acid."

By a "therapeutic composition" is meant a composition appropriate for administration to an animal, for example, a mammal, such as a human, a livestock species (for example, a bovine, goat, pig, or sheep), or a pet species.

By a "small molecule" is meant a compound with a molecular weight of less than or equal to 10,000 Daltons, preferably, less than or equal to 1000 Daltons, and, most preferably, less than or equal to 500 Daltons.

Other features and advantages of the invention will be apparent from the following description of the preferred embodiments thereof, and from the claims.

### Brief Description of the Drawings

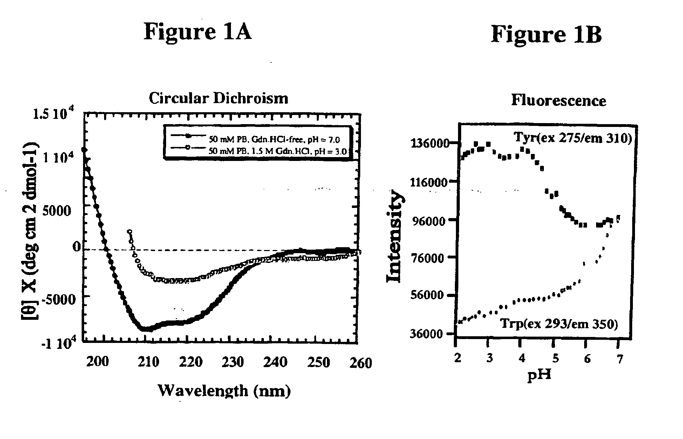

Figure 1A is a graph showing circular dichroism of pH-related conformational changes in mouse recombinant PrP^{C}. Far-UV circular dichroism spectroscopy of PrP^{C} at pH of 7.0 and 3.0 shows a shift in molecular ellipticity consistent with a change in conformation from predominantly alpha helix at physiological pH to predominantly beta sheet at low pH.
Figure 1B is a plot showing the fluorescence spectroscopy of pH-related changes in aromatic ring orientation in mouse recombinant PrP^{C}. Changes in specific fluorescence of tyrosine and tryptophan side chains are consistent with opposing tendencies for solvent accessibility as pH is reduced.
Figure 2 shows the alignment of PrP amino acid sequences from bovine (SEQ ID NO: 26), man (SEQ ID NO: 27), sheep (SEQ ID NO: 28), mouse (SEQ ID NO: 29), and hamster (SEQ ID NO: 30). The boxes highlight the two highly conserved YYR sequences at amino acids 160-162 and 173-175, as well as the YYQ/D sequence at amino acids 235-237 (according to the bovine sequence).

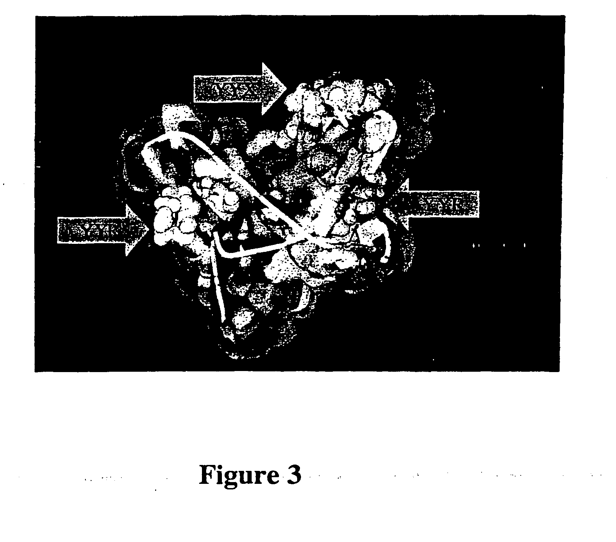

Figure 3 shows an NMR solution structure of mouse PrP^{C}, highlighting the location and orientation of the YYR and YYD sequences. None of the YY motifs show the so-called orthogonal tandem orientation of aromatic rings to the molecular surface.

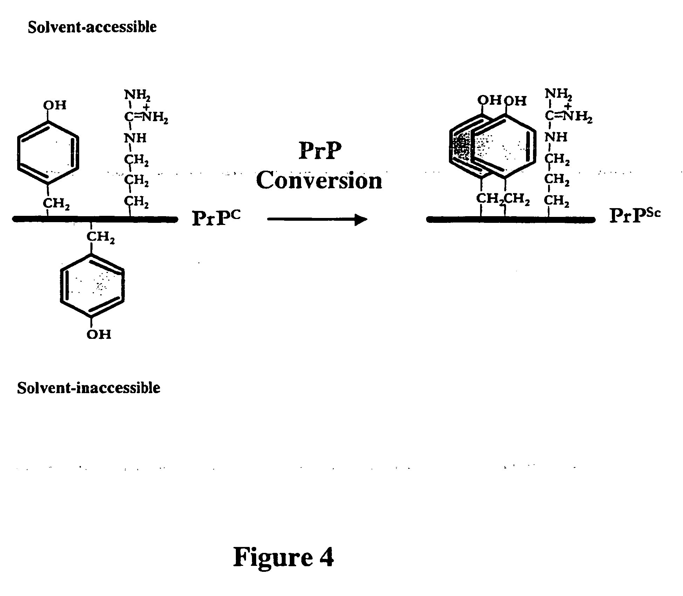

Figure 4 is a schematic showing epitope generation based on a change in tyrosine ring orientation that occurs with conformational change of PrP^{C} to PrP^{Sc}. As depicted in Figure 3, two YYR motifs in PrP^{C} are oriented such that one tyrosine ring is solvent-accessible, whereas the other is solvent-inaccessible in the molecular interior. (The third YY motif, not shown in this schematic, is oriented such that the tyrosine rings are unable to interact). Upon PrP conformational conversion, tyrosine rings in one or more YYX motif become accessible at the molecular surface in an orthogonal tandem orientation that is stabilized by pi-stacking interactions. The terminal planar arginine (or aspartate/glutamine in the third motif) may additionally stabilize the tyrosine-tyrosine interaction.

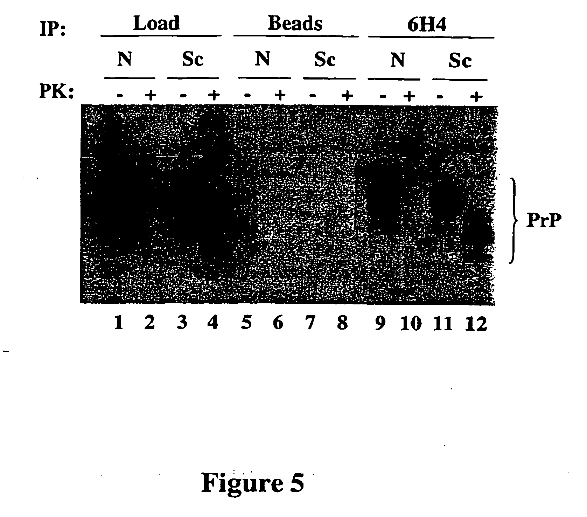

Figure 5 shows the immunoprecipitation (IP) of mouse PrP^{Sc} using magnetic beads conjugated to control proteins. Non-distinguishing PrP monoclonal antibody 6H4 coupled to magnetic beads (lanes 9-12) or beads coupled to BSA (beads, lanes 5-8) were reacted with normal (N) or ME7 scrapie infected (Sc) mouse brain homogenates. The brain homogenates were treated with proteinase K(+) or not (-) prior to the immunoprecipitation. In lanes 1-4 (Load) are displayed direct western blots of the same amount of brain homogenates that were used in the immunoprecipitations. The immunoprecipitates were resolved on SDS-PAGE gels under non-reducing conditions. Blots were probed with 6H4 monoclonal antibody followed by goat anti-mouse Ig-HRP conjugate.

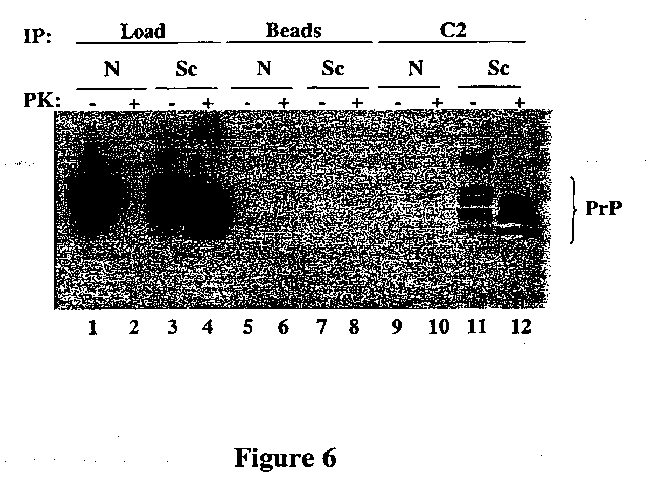

Figure 6 shows the immunoprecipitation (IP) of mouse PrP^{Sc} using magnetic beads conjugated with the anti-YYR polyclonal antibody pAbC2. pAbC2-coupled magnetic beads (lanes 9-12) or beads coupled to BSA (beads, lanes 5-8) were reacted with normal (N) or ME7 scrapie infected (Sc) mouse brain homogenates. The brain homogenates were treated with proteinase K (+) or not (-) prior to the immunoprecipitation. In lanes 1-4 (Load) are displayed direct western blots of the same amount of brain homogenates that were used in the immunoprecipitations. The immunoprecipitates were resolved on SDS-PAGE gels under non-reducing conditions. Blots were probed with 6H4 monoclonal antibody followed by goat anti-mouse Ig-HRP conjugate.

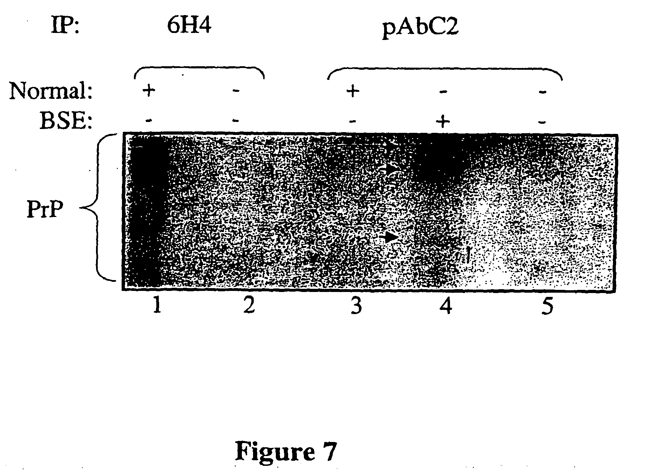

Figure 7 shows the immunoprecipitation of bovine PrP^{Sc} using magnetic beads conjugated with pAbC2 rabbit polyclonal antibody. 6H4 (lanes 1 and 2) or pAbC2-coupled magnetic beads (lanes 3 and 4) were used to immunoprecipitate normal or BSE brain homogenates. The immunoprecipitates were resolved on SDS-PAGE gels under non-reducing conditions. Blots were probed with 6H4 monoclonal antibody followed by goat anti-mouse Ig-HRP conjugate.

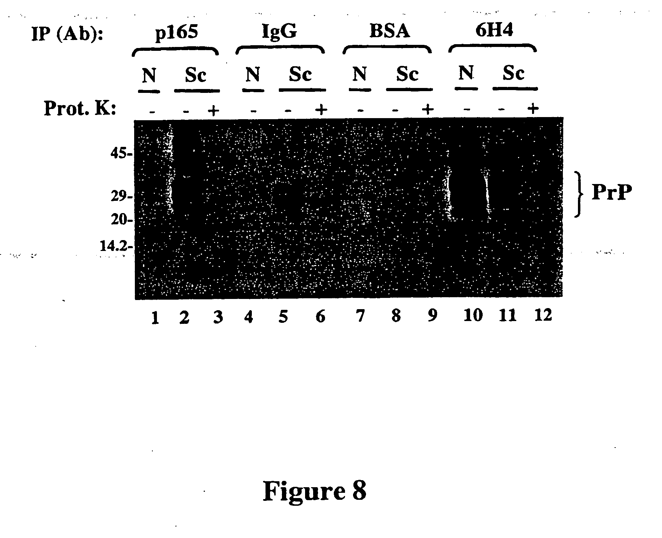

Figure 8 shows the immunoprecipitation of mouse PrP^{Sc} using magnetic beads conjugated with the anti-YYR p165 goat polyclonal antibody. Antibody-coupled magnetic beads were reacted with normal (N) or ME7 scrapie infected (Sc) mouse brain homogenates. The scrapie samples were treated with proteinase K (+) or not (-) prior to the immunoprecipitation. Lanes 1-3 (p165) affinity-purified goat pAb; Lanes 4-6 (IgG) total goat IgG; Lanes 7-9 (BSA) BSA-conjugated beads; Lanes 10-12 (6H4) non-discriminating anti-PrP monoclonal antibody 6H4. The immunoprecipitates were resolved on SDS-PAGE gels under non-reducing conditions. Blots were probed with 6H4 monoclonal antibody followed by goat anti-mouse Ig-HRP conjugate.

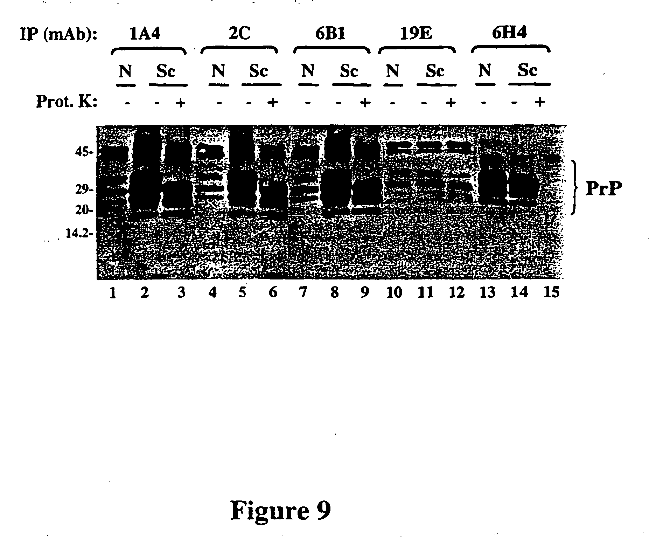

Figure 9 shows the immunoprecipitation of mouse PrP^{Sc} using magnetic beads conjugated to anti-YYR mouse monoclonal antibodies. Monoclonal antibody-coupled magnetic beads were reacted with normal (N) or ME7 scrapie infected (Sc) mouse brain homogenates. The scrapie samples were treated with proteinase K (+) or not (-) prior to the immunoprecipitation. 1A4 (lanes 1-3), 2C (lanes 4-6), and 6B1 (lanes 7-9) are scrapie reactive monoclonal antibodies. 19E (lanes 10-12) is a monoclonal antibody unreactive with PrP. 6H4 (lanes 13-15) is an anti-PrP monoclonal antibody that does not discriminate PrP^{C} from PrP^{Sc}. The immunoprecipitates were resolved on SDS-PAGE gels under non-reducing conditions. Blots were probed with 6H4 monoclonal antibody followed by goat anti-mouse Ig-HRP conjugate. In lanes 10-12, corresponding to 19E, immunoglobulin heavy (45 kDa) and light (30 kDa) chain from the antibody-bead conjugates co-migrate with PrP dimers, and one of the PrP glycosylation variants, respectively.

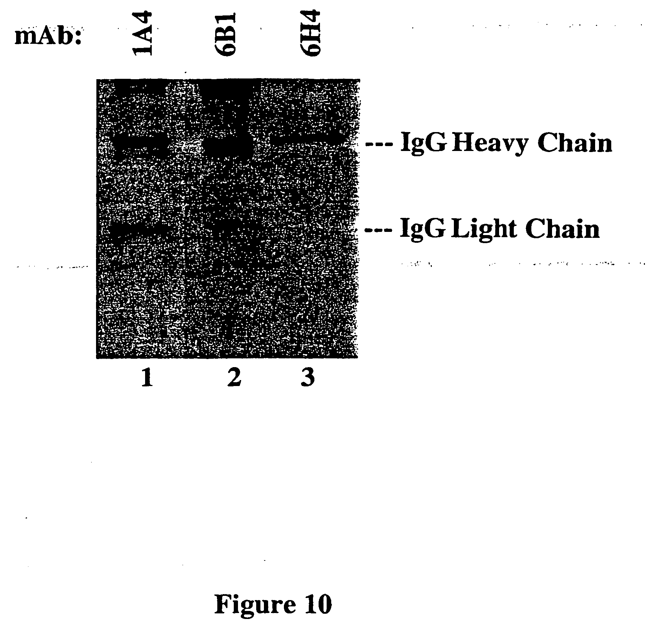

Figure 10 shows the immunoglobulin light chain and heavy chain leakage from the antibody-bead conjugates. Monoclonal antibody 1A4 and 6B1 magnetic beads conjugates were processed for SDS PAGE and western blot analysis in the absence of brain homogenates. SDS-PAGE gels were run under non-reducing conditions for these two antibody-bead conjugates and under reducing conditions for the unconjugated 6H4 monoclonal antibody. Blots were developed with goat anti-mouse Ig-HRP conjugates.

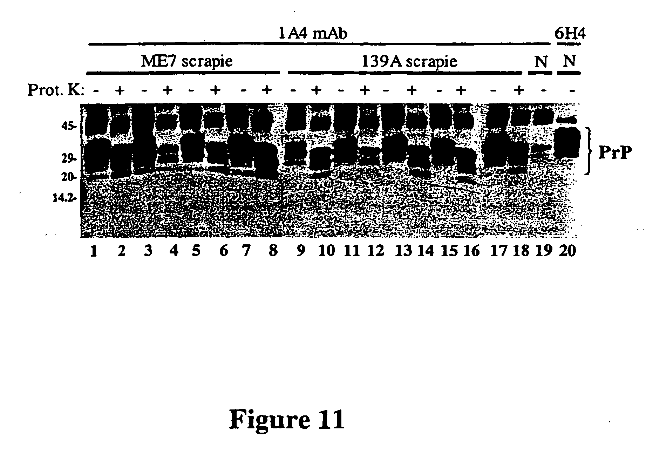

Figure 11 shows the immunoprecipitation of multiple mouse PrP^{Sc} samples using magnetic beads coupled with the anti-YYR 1A4 monoclonal antibody. 1A4-magnetic bead conjugates were reacted with normal (N, lane 19), four ME7 (lanes 1-8), or five 139A (lanes 9-18) scrapie infected mouse brain homogenates. The scrapie samples were treated with proteinase K (+) or not (-) prior to the immunoprecipitation. 6H4-magnetic bead conjugates were reacted with normal (N, lane 20) mouse brain homogenates as a control. The immunoprecipitates were resolved on SDS-PAGE gels under non-reducing conditions. Blots were probed with 6H4 monoclonal antibody followed by goat anti-mouse Ig-HRP conjugate.

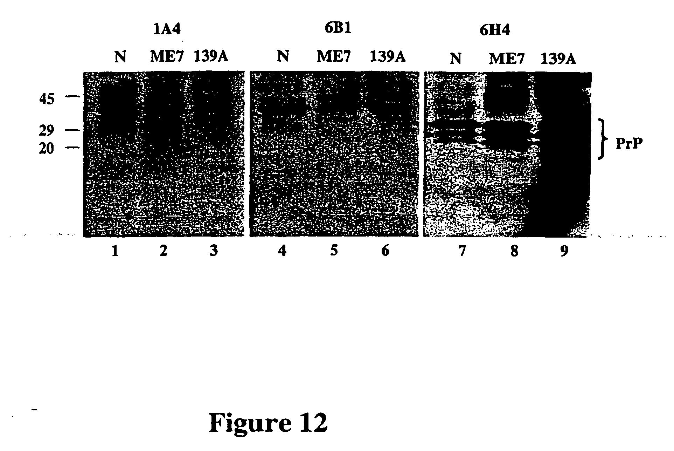

Figure 12 shows the conformation dependence of anti-YYR monoclonal antibody reactivity to PrP^{Sc}. Normal (N), ME7, and 139A scrapie infected mouse brain homogenates were resolved in SDS-PAGE gels under non-reducing conditions. Blots were probed with 1A4 (lanes 1-3) or 6B1 (lanes 4-6) followed by goat anti-mouse Ig-HRP conjugates. The blots were then re-probed, without stripping, using 6H4 (lanes 7-9) and goat anti-mouse Ig-HRP conjugates.

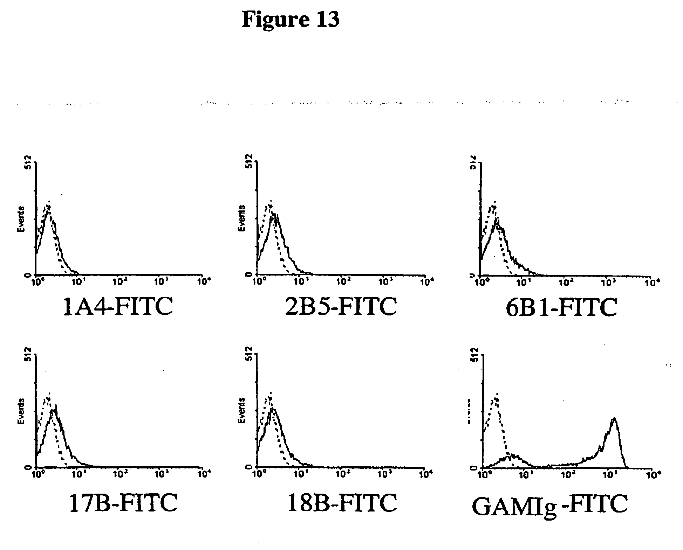

Figure 13 shows an analysis of dissociated normal mouse splenocytes reacted with fluoresceinated anti-PrP^{Sc}monoclonal antibodies 1A4, 2B5, 6B1, 17B, and 18B using flow cytometry. Fluoresceinated goat anti-mouse Ig (GAMIg) was used as a control. Cells were gated on acquisition by characteristic forward and side scatter parameters and for exclusion of propidium iodide. The dashed lines represent background fluorescence; solid lines represent antibody staining. The monoclonal antibodies were successfully fluorescein labelled as measured by fluorescein emission in a 96-well plate reader and maintained their reactivity towards the immunizing antigen by ELISA.

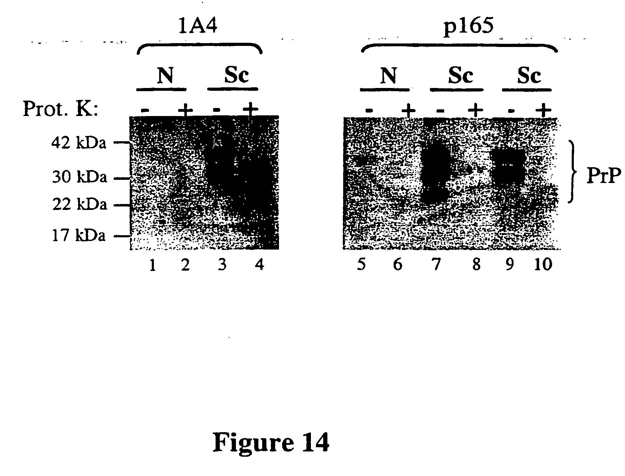

Figure 14 shows the immunoprecipitation of hamster PrP^{Sc} using anti-YYR mouse monoclonal antibody and goat polyclonal antibody. 1A4 monoclonal antibody and p165 polyclonal antibody-magnetic bead conjugates were reacted with normal (N) or scrapie infected hamster brain homogenates (Sc). The scrapie samples were treated with proteinase K (+) or not (-) prior to the immunoprecipitation. The immunoprecipitates were resolved on SDS-PAGE gels under non-reducing conditions. Blots were probed with 6H4 monoclonal antibody followed by goat anti-mouse Ig-HRP conjugate.

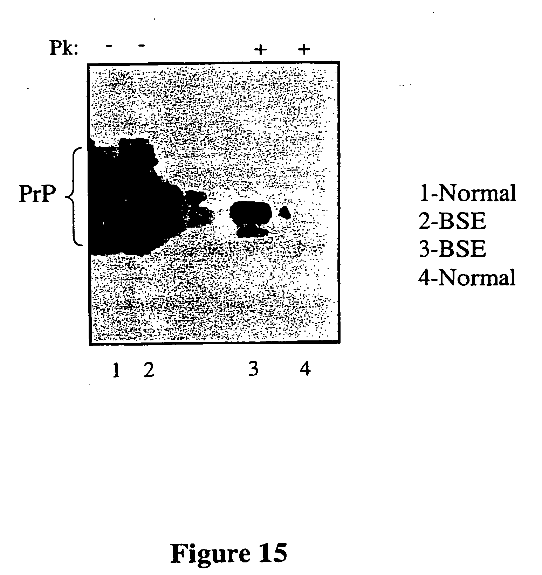

Figure 15 shows an immunoblot detection of protease-sensitive PrP^{C} in normal bovine brain (lanes 1 and 4), and PrP^{C} and protease-resistant PrP^{Sc} in BSE brain (lanes 2 and 3). Brain homogenates treated or not treated with proteinase K (+ or -) were resolved on SDS-PAGE electrophoresis, and transferred onto a PVDF membrane for immunblotting. The membrane was probed with mAb 6H4, washed, and developed with ECL and exposed to X-ray film according to standard procedures.

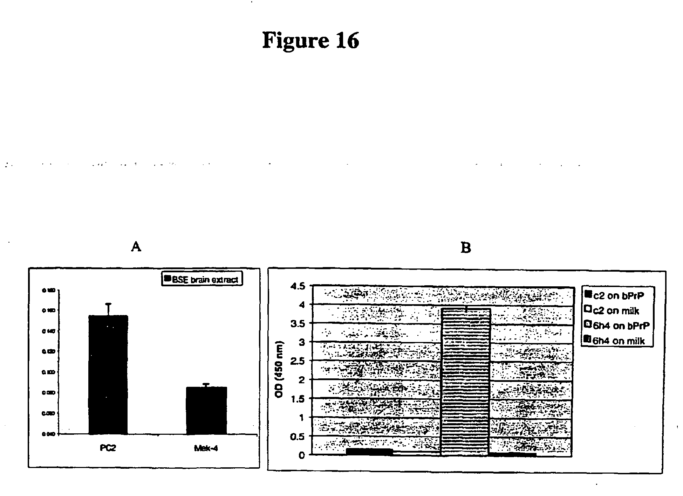

Figure 16 shows ELISA systems demonstrating specific bovine PrP^{Sc} recognition by the anti-YYR rabbit polyclonal antibody pAbC2. BSE brain extract reacted with ELISA-adsorbed soluble prion receptor ectodomain (PC2) or control protein (Mek) showed statistically significant detection by pAbC2 of bound PrP^{Sc} in receptor vs control wells (Panel A; p-value=0.008). The recognition of recombinant bovine PrP^{C} (bPrP) by direct ELISA with pAbC2 or mAb6H4 is also shown (Panel B).

### Detailed Description of the Invention

We have determined that the orientation of selected aromatic side chains of tyrosines of a YYX epitope (e.g., YYR) at one or more sites in PrP defines a continuous immunologic epitope specific for the molecular surface of PrP^{Sc}, whereas the same tyrosine side chains are known to be inaccessible in the PrP^{C} conformation, according to published PrP^{C} NMR structure solutions (Riek et al., *Nature* 382:180, 1996; Donne et al., *Proc. Natl. Acad. Sci. USA,* 94:13452-7, 1997; Zahn et al, *Proc. Natl. Acad. Sci. U S A,* 97:145-50, 2000). This discovery facilitates the generation of PrP^{Sc}-specific antibodies which may be used for diagnostic and therapeutic purposes, as well as the development of screens for novel compounds useful to detect or combat prions and their related diseases and disorders.

### Generation of Epitope-Specific Antibodies to PrP^{Sc}

Antibodies specifically recognize proteins via unique amino acid determinants or epitopes. These determinants or epitopes may be of a linear amino acid sequence or distinct conformations formed by amino acids in three-dimensional space. Considering conversion of PrP^{C} to PrP^{Sc} involves a major change in protein conformation, it is likely that unique epitopes will be formed or revealed upon conversion. Therefore, as is discussed herein, we have developed a so-called side chain hypothesis pertaining to prion protein conversion. According to this scheme, side chains normally sequestered in the solvent-inaccessible interior of PrP^{C} may be solvent accessible in PrP^{Sc}. The preponderance of newly exposed side chains are therefore expected to be hydrophobic, as evidenced by increased solvent exposure of hydrophobic residues in a stable PrP^{Sc}-like intermediate (Swietnicki et al, *J. Biol. Chem.* 272:27517-20, 1997). The extrusion of these hydrophobic side chains, alone or in combination with side chains that are normally present on the molecular surface of PrP^{C}, form the basis of unique epitopes for antibody recognition of PrP^{Sc}. Moreover, these surface-accessible hydrophobic side chains are expected to change the solubility and aggregation characteristics of PrP^{Sc}, commensurate with the known properties of this structural isoform. Newly accessible side chains may also participate in the process of recruitment of PrP^{c} to PrP^{Sc}.

Testing this hypothesis began *in vitro* by examining the orientation of tryptophan and tyrosine rings, two hydrophobic amino acid side chains for which information was obtained by fluorescence spectroscopic studies. First, a beta sheet transition of mouse recombinant PrP^{C} was induced by low pH in order to model the structural changes that characterize the conversion of PrP^{C} to PrP^{Sc} (Hornemann and Glockshuber, *Proc. Natl. Acad. Sci.* **95:**6010, 1998). Figure 1A demonstrates a shift in molecular ellipticity by circular dichroism from a pH of 7.0 to 3.0 consistent with a change of PrP^{C} from predominantly alpha helix to predominantly beta sheet (spectra shifting from double to single minima at appropriate respective frequencies).

Second, the solvent accessibility of tyrosine and tryptophan side chains in recombinant PrP^{C} with pH titration was examined using standard fluorescence spectroscopy (Figure 1B). This study is based on the principle that aromatic side chains which are neighboring other amino acid side chains (i.e., in the interior of the protein) will possess a different specific fluorescence than aromatic side chains exposed to water (for example, Chin et al, *Biochemistry* 31:1945-51,1992). When recombinant mouse PrP^{C} was subjected to low pH, tryptophan and tyrosine aromatic groups displayed opposing behaviors consistent with differential solvent exposure.

Inspection of the amino acid sequence of human, bovine, and murine PrP^{C} revealed thirteen tyrosine residues (Figure 2). Eleven tyrosines in human and bovine PrP and 10 in murine PrP are contained in the C-terminal 2/3 of the protein, which comprises the protease-resistant structured domain necessary and sufficient for prion infectivity. Remarkably, six tyrosines in this domain are present in the unusual "YY" paired motif (Figure 2). Two of the three pairs are in conjunction with a C-terminal arginine (R), whereas the third YY motif is in conjunction with a C-terminal aspartate (D) in mice and hamsters or a glutamine (Q) in cattle, sheep, and humans. R contains a terminal guanido group, whereas Q and D contain carboxamide and carbonyl bonds, which are planar. Such a terminal planar amino acid in the YY motif may interact with the exposed tyrosine rings to stabilize or shepherd them for immune recognition.

In addition, inspection of the NMR-resolved structures of murine, hamster, and human PrP^{C} revealed that none of the identified tyrosine pairs are oriented with both of their rings in an orthogonally tandem configuration accessible on the molecular surface (Riek et al., *Nature,* 382:180, 1996; Donne et al., *Proc. Natl. Acad. Sci. ,* 94:13452, 1997; Zahn et al, *Proc. Natl. Acad. Sci. U S A,* 97:145-50, 2000) (Figure 3). It is reasonable to surmise that the increased exposure of tyrosine on the surface of acid-treated PrP^{C} or PrP^{Sc} is associated with some of the tyrosine pairs. One stable conformation of tyrosine rings is referred to as pi-stacking, in which the two rings are stacked in slightly displaced parallel manner (schematically illustrated in Figure 4). Although stable, a preliminary search of the structural databases for pi-stacked surface accessible tyrosine rings identified only 4 other proteins with similar orientation, none of which are in the ectodomain of membrane proteins. Therefore, a novel PrP^{Sc} - specific epitope is thought to be tyrosine pairs in a pi-stacking orientation, with or without the contribution of side chains of arginine, glutamine, and aspartate (which, being planar, may also participate in a pi-stacking interaction with its preceding tyrosine).

In addition to changes in orientation of tyrosine side chains in PrP^{Sc}, it is also possible that the three YYR motifs become more immunologically accessible in PrP^{Sc} because of shifts in their proximity to each other. A typical IgG antibody is comprised of two identical antigen-binding regions that are connected to one constant region by a flexible hinge region. During the conformational change of PrP^{C} to PrP^{Sc}, YYR motifs probably move relative to each other (Korth et al., *Nature,* 390:74-77, 1997), moving from relatively separated to relatively close. In addition, IgG recognition of YYR motifs in PrP^{C} may be unfavorable because two critical motifs are on different sides of the molecule, rendering the recognition to be of a low-avidity univalent nature, rather than the high-avidity interaction in which both IgG antigen binding regions participate in recognition.

Our data with bovine PrP^{C} and PrP^{Sc} showed that anti-YYR antibodies specifically recognize PrP^{Sc} by immunoprecipitation and ELISA testing (see below), consistent with changes in the accessibility of tyrosine side chains in PrP^{Sc} and/or proximity of the YYR epitopes. The amino acid residue, arginine, is also thought to be important in the generation and recognition specificity of the YYR antibody. It is believed that electrostatic interaction between polar tyrosine side chains and the highly basic side chain of arginine contribute to the nature of the YYR epitope in both immunization by the YYR tripeptide, and the recognition of PrP^{Sc} by the derived antibody. It is notable that the third YY dimer motif in the terminal PrP loop is associated with a glutamine in some species (including humans and cattle), which is a partially conservative substitution with arginine, and aspartate in some other species (including mice and hamsters), which is not a conservative substitution. Exemplary amino acids having planar side chains include arginine, aspartate, and glutamine.

The phenomenon of amino acid side chain exposure incident on conformational conversion of PrP^{C} to PrP^{Sc} may not uniquely apply to tyrosine pairs. It is possible that other amino acids with bulky side chains may find these side chains to be poorly tolerated in the core of PrP^{Sc}, and that these side chains, alone or in combination with other local moieties, form the basis of unique immunoreactivity of PrP^{Sc}. Immunological epitopes differing between PrP^{C} and PrP^{Sc} form the basis of a diagnostic test for PrP^{Sc}, and are also useful in the treatment and immunization of humans and animals against prion disease. Hydrophobic side chain exposure is thought to be responsible for the increased hydrophobicity and enhanced aggregation of PrP^{Sc} compared to PrP^{C}.

Examples of bulky side chains not fully accessible to solvent in PrP^{C} include the following (amino acid residues are numbered according to the mouse PrP sequence):
1. Tyrosines not contained in the YYX motif, including Y127, Y156, Y217.
2. A threonine tetrarepeat, T189-193, partially not exposed to solvent.
3. Histidine H 186.
4. Glutamine Q159, Q167, Q185, Q216.
5. Asparagine N158.
6. Methionine M128, M 133, M 153.
7. Leucine L124, L129.
8. Isoleucine I181, I183.

As is described below, peptides containing the YYR epitope were synthesized. These peptides were conjugated to a carrier and used to immunize rabbits or mice for the production of polyclonal or monoclonal antibodies. Polyclonal and monoclonal antibodies were then tested for specificity to PrP^{Sc}. The results indicated that such antibodies bound specifically, with high binding affinity to PrP^{Sc}.

The following examples described below are provided for the purpose of illustrating the invention, and should not be construed as limiting.

### PrP^{Sc}-Specific Antibodies

Polyclonal antisera, pAbC2, were raised in rabbits against a YYR peptide linked to KLH. Serum was collected from each rabbit after the immunization regime and total IgG purified using a Protein A column. Anti-PrP^{Sc} activity of these samples was then tested in immunoprecipitation reactions using brain homogenates from normal or scrapie-infected mice. Initial analysis of the brain homogenates used in these studies revealed detectable amounts of PrP^{C} in normal brain extracts (Figure 5, lane 1) and PrP in infected samples (Figure 5, lane 3). As expected, PrP^{C} was sensitive to digestion by proteinase K (PK) (Figure 5, lane 2), whereas the characteristic migration shift of the protease-resistant core of PrP^{Sc} (designated PrP 27-30) was evident upon PK digestion (Figure 5, lane 4). Incubation of these brain homogenates with BSA-coupled magnetic beads failed to precipitate any detectable PrP (Figure 5, lanes 5-8), whereas, incubation of the samples with beads coupled with 6H4 (a PrP-specific monoclonal antibody) immunoprecipitated PrP^{C} from normal brains (Figure 5, lane 9), and PrP^{Sc} (Figure 5, lane 11) and PrP 27-30 (Figure 5, lane 12) from infected brains. When pAbC2 IgG was coupled to the beads and incubated with normal brain homogenates, no detectable PrP was immunoprecipitated (Figure 6, lanes 9 and 10). Strikingly, incubation of the pAbC2 IgG-coupled beads with infected samples immunoprecipitated PrP^{Sc} (Figure 6, lane 11) and PrP 27-30 (Figure 6, lane 12). Once again, these tissues harbored detectable amounts of PrP^{C} and PrP^{Sc} (Figure 6, lanes 1-4) and BSA-coupled beads failed to immunoprecipitate any PrP (Figure 6, lanes 5-8).

In addition, similar experiments showed that pAbC2 IgG specifically immunoprecipitates bovine PrP^{Sc} from BSE infected brains compared to 6H4 (Figure 7). Moreover, pAbC2 antibodies can recognize bovine PrP^{Sc} in an ELISA system using soluble PC2 (prion receptor) as a capture reagent, but generates no signal in ELISA studies in which recombinant bovine PrP^{C} is directly adsorbed to plates, despite its detectability using the 6H4 monoclonal antibody (Figure 16). Anti-YYR IgG does not recognize denatured recombinant bovine PrP^{C} in western blotting (data not shown), similar to studies detailed below with mouse anti-YYR monoclonal antibodies.

A goat polyclonal antisera was also raised to YYR linked to KLH. Serum was collected and total IgG was isolated by ammonium sulfate precipitation. YYR-reactive IgG was also purified from the same sera by affinity chromatography using a YYR-conjugated column. These antisera were subjected to a similar set of screening and validating immunoprecipitation reactions as detailed above. One of the three immunized goats developed antiserum (p165) that was specific for PrP^{Sc} (Figure 8) and was further characterized. As in previous experiments, 6H4-coupled beads non-discriminately precipitated both PrP^{C} and PrP^{Sc} from infected mouse brains (Figure 8, lanes 10 and 11). No 6H4 immunoprecipitation of PrP 27-30 was seen in this experiment (Figure 8, lane 12), as is occasionally observed for unknown reasons. When total IgG from immunized goats was coupled to the beads, little, if any, material was precipitated from normal and infected mouse brain homogenates (Figure 8, lanes 4, 5 and 6). In contrast, when YYR-affinity purified IgG (p165) was coupled to the magnetic beads, only PrP^{Sc} was precipitated from infected mouse brains (Figure 8, lane 2). Unlike the pAbC2 polyclonal from rabbits, the goat anti-YYR polyclonal did not precipitate PrP 27-30 (Figure 8, lane 3). These extracts contained detectable PrP 27-30 following PK digest, as simultaneous experiments evaluating monoclonal antibodies (see below) used the same homogenates and PrP 27-30 was detected upon PK digestion (Figure 9, lanes 3, 6 and 9). Moreover, analysis of the brain homogenates by western blot revealed detectable PrP^{Sc} and PrP 27-30 in the brain homogenates from infected mice (data not shown).

In addition to the goat polyclonal antibody generation, monoclonal antibodies against the same PrP^{Sc}-specific epitope were also generated, but with a derivative of the original antigen in which multiples of the original YYR peptide were linked together into one contiguous sequence. YYRRYYRYY (SEQ ID NO: 31) was synthesized in an attempt to increase the number of YYR epitopes in the peptide sequence, and to increase the chance of tyrosine stacking and/or frequency of pi-stacking. Moreover, one of the YYR sequences in the prion protein is preceded by an arginine in the five species of interest (Figure 2). The YYRRYYRYY peptide was linked to KLH and mice were subsequently immunized with the antigen. Splenocytes from these mice were isolated and fused to the FO murine B cell line (ATCC CRL-1646) to generate specific hybridoma clones. Ascities were produced from clones that reacted with YYR conjugated to an alternative carrier, 8map, in an ELISA. IgG from these ascities were purified using a Protein-A column and screened and validated using standard methods. Five monoclonal antibodies were identified that specifically recognized PrP^{Sc} in immunoprecipitation reactions using brain homogenates from infected mice (1A4, 6B1, 2B5, 2C, and 18B). Figure 9 illustrates the specific precipitation of PrP^{Sc} for three of these monoclonal antibodies, 1A4, 2C, and 6B1. As was seen for p165, 1A4, 2C, and 6B1 specifically precipitated PrP^{Sc} (Figure 9, compare lanes 1, 3 and 5 with lanes 2, 4 and 6 respectively) compared to a negative control antibody, 19E (Figure 9, lanes 10 and 11), and the non-distinguishing antibody 6H4 (Figure 9, lanes 14 and 15). In contrast to p165, all three of these PrP^{Sc}-specific antibodies precipitated PrP 27-30 (Figure 9, lanes 3, 6 and 9). The faint bands present in the 1A4, 2C and 6B precipitations using the normal mouse brain homogenates (Figure 9, lanes 1, 3 and 5) and in the precipitations with 19E probably represent a combination of nonspecific background precipitation of PrP^{C}, and the murine IgG light and heavy chains eluted from the magnetic beads that were subsequently detected by goat anti-mouse Ig-HRP conjugate (Figure 10).

Continued evaluation of the PrP^{Sc}-specific monoclonal antibodies revealed that they were capable of recognizing different murine strains of PrP^{Sc} through a conformationally dependent epitope. As depicted in Figure 11, 1A4 was used in immunoprecipitations on numerous extracts prepared from different mice infected with either the ME7 or 139A strain of murine scrapie. In these experiments, 1A4 was found to specifically precipitate PrP^{Sc} and PrP 27-30, regardless of the strain. This was also found for the other PrP^{Sc}-specific monoclonal antibodies (data not shown). When the brain homogenates (normal, ME7 and 139A infected) were electrophoresed in an SDS-PAGE gel under non-reducing conditions and then probed for PrP with one of the PrP^{Sc}-specific monoclonal antibodies (1A4 or 6B1) or 6H4, it was clearly evident that only 6H4 was capable of detecting denatured PrP^{C} and PrP^{Sc} (Figure 12, lanes 7, 8 and 9). For 1A4 and 6B1, the PrP^{Sc}-specific determinants had been lost upon denaturation of the sample, establishing the conformational sensitivity of the epitope.

In addition, it was determined that YYR-reactive monoclonal antibodies do not recognize any cell surface proteins on the surface of splenocytes or dissociated immediately *ex-vivo* brain cells from normal and PrP-/- knockout mice. Viable mouse splenocytes and brain cells were isolated by centrifugation of spleen cell and brain suspensions through a ficol gradient. As shown in Figure 13, splenocytes were stained with the FITC-conjugated antibodies listed (solid lines), or with isotype-matched FITC labeled control antibodies (dashed lines). Non-viable cells were excluded from the analysis with propidium iodide. The lack of surface immunoreactivity on splenocytes (Figure 13) or brain cells (not shown) indicates that the YYR conformational epitope is rare, as suggested by the structural searches noted above. Moreover, the lack of appreciable signal provides an acceptable background for studies of PrP^{Sc} immunoreactivity at the cell surface of splenocytes and other test cells. Detection of cell surface PrP^{Sc} is useful as a diagnostic test for human and animal prion disease infection. Finally, the lack of cell surface immunoreactivity provides an independent verification of the fact that anti-YYR antibodies do not recognize PrP^{C}, as splenocytes and brain cells possess detectable surface PrP^{C} by 6H4 immunohistochemistry (not shown).

Species specificity of the YYR epitope has also been examined. In studies using brain homogenates from scrapie-infected hamsters, the above-described PrP^{Sc}-specific monoclonal and polyclonal antibodies were found to immunoprecipitate hamster PrP^{Sc} (Figure 14). In addition, similar specificities for PrP^{Sc} and PrP 27-30 that were evident in the studies with infected murine tissues were also observed. For example, monoclonal antibody 1A4 specifically immunoprecipitates PrP^{Sc} and PrP 27-30 (Figure 14, lanes 3 and 4), whereas the polyclonal goat antibody p165 specifically immunoprecipitates only PrP^{Sc} from infected hamster brains (Figure 14, lanes 7, 8, 9, and 10). In addition to hamster, PrP^{Sc} from infected sheep, bovine, and human tissues, if desired, may be specifically precipitated using any of the techniques described herein.

### Materials and Methods

These PrP^{Sc}-specific antibodies described above were obtained and tested using the following materials and methods.

### Circular Dichroism

Circular dichroism was performed according to methods described by Hornemann and Glockshuber (*Proc. Natl. Acad. Sci.* 95:6010, 1998). Far-UV circular dichroism spectra were recorded on an Aviv Circular Dichroism Spectrometer model 62DS (Lakewood, NJ) at 25°C using quartz cells with a path length of 0.1 cm. Spectra were obtained from 195 nm to 260 nm, with a 1.0 nm step, 1.0 nm bandwidth, and 4-second collection time per step. The experimental data were expressed as mean residue ellipticity (deg θcm²dmol⁻¹).

### Fluorescence Spectroscopy

Fluorescence spectroscopy was performed according to the methods described in Chin et al. (*Biochemistry* 31:1945-51, 1992).

### Preparation of the immunizing YYR peptides

In order to develop an antibody to PrP^{Sc}, a peptide with the amino acid sequence Acetyl -Cys-Tyr-Tyr-Arg-NH2 (YYR) (SEQ ID NO: 32) was synthesized, conjugated to KLH, and injected intramuscularly into rabbits using well known techniques.

At the amino-terminus of the peptide, a cysteine residue was added to allow conjugation of the peptide with the protein carrier. The amino group of the peptide was blocked by acetylation, and the carboxylic group of the peptide was blocked by amidation.

### Peptide Synthesis

Peptides were synthesized using solid phase peptide synthesis methods either manually or automated (MPS396 peptides synthesizer, Advanced ChemTech). Coupling of amino acid residues was accomplished using Fmoc peptide synthesis chemistry (Fields et al., 1990, *IJPPR* 35, 161). Syntheses were performed on Wang or on amide Rink resins, with full side chain protection of amino acids. Since the alpha-NH₂ groups of the amino acids were protected with the Fmoc group, the following protective groups were chosen for the side groups of the trifunctional amino acids:

| | |
|---|---|
| Cysteine: | 5-triphenylmethyl (Trt) |
| Arginine: | 2,2,4,6.7-pentamethyldihydrobenzofuran-5-sulfonyl (Pbf) |
| Tyrosine: | tert.-butyl ether (tBu) |

BOP, PyBOP, or TBTU were used as activation agents, depending on the chemistry and difficulty of the coupling reaction. All chemicals were purchased from Advanced ChemTech, Bachem, and Calbiochem/NovaBiochem. Formation of each peptide bond between residues of the sequence was ensured by using a 3 to 6 fold excess of coupling reagents and by so-called double coupling; meaning that the coupling reaction was repeated for each amino acid added to the growing peptide chain.

### Cleavage of fluo-peptides from resin

After synthesis, the peptides were cleaved from the resin using the Reagent K as a cleavage mixture: water (2.5%), TIS (2.5%), EDT (2.5%), TFA (92.5%). The peptides were then precipitated with cold diethyl ether. The precipitates were centrifuged, washed three times with diethyl ether, dissolved in 20%-50% AcCN/water mixture, and lyophilized. Analysis of crude products was performed using analytical RP-HPLC and electrospray MS.

### HPLC Purification

The crude peptide was purified by RP-HPLC (reverse phase high performance liquid chromatography) on a Vydac C18 column, 2.5x25 cm, using a linear gradient of 10-50% acetonitrile in water, with 0.06% TFA (1%/min gradient, 10 ml/min flow rate), with monitoring by UV at 215 nm and 254 nm. Analytical HPLC was used to estimate the purity of the fractions. The final product was obtained as a lyophilized peptide with at least 95% purity estimated by analytical HPLC (Vydac C18, 0.46x25 cm, linear gradient 10-60% acetonitrile in water, 0.1% TFA, 1%/min, 1ml/min flow rate, detection by UV absorption at 215 nm and 254 nm). The pure peptide was identified by molecular mass analysis using a SCIEX API III mass spectrometer according to standard procedures.

### Analytical Data

The retention time of the peptide on RP-HPLC was 21.215 minutes. The theoretical molecular weight of the peptide was calculated to be 644.74; the actual molecular weight, through molecular mass analysis, was found to be 646.5 (MW+H⁺).

### Coupling of the peptide to a carrier

Peptides were coupled to a carrier, in this case keyhole limpet hemocyanin (KLH). Other carriers useful for such coupling include, without limitation, albumin, or ovalbumin, 8map, or lysozyme. Coupling was effected via a thioether linkage to the mercapto group of the cysteine. This type of linkage has the advantage that the peptide is coupled in a defined way to a carrier protein.

Coupling to KLH was performed as follows. 10 mg of the peptide was dissolved in 2 ml of phosphate buffered solution (PBS 1x). 1 ml of KLH (Pierce products #77100) was added to the peptide solution and stirred (1 mole of peptide/50 amino acids). The KLH concentration was 10 mg/ml. 20 µl of glutaraldehyde (25% aqueous solution) was added to the peptide/carrier solution with constant stirring, incubated for 1 hour, after which a glycine stop solution was added. The peptide/carrier conjugate was separated from the peptide by dialysis against PBS.

Additional YYR peptides (e.g., CYYRRYYRYY (SEQ ID NO: 33) and CKYEDRYYRE (SEQ ID NO: 34)) were synthesized according to standard methods, for example, those described herein. Other synthetic peptides can be prepared by making appropriate modifications of the above- described synthetic methods. Such peptides are also characterized using any of the standard methods known in the art (e.g., those described herein).

### Immunization of rabbits

Polyclonal antibodies were prepared according to standard methods, and an immune response was enhanced with repeated booster injections, at intervals of 3 to 8 weeks. The success of the immunization was verified by determining the concentration of antibodies in a western blot or ELISA or both. More specifically, to generate polyclonal antibodies to PrP^{Sc}, the tripeptide YYR conjugated to KLH was injected into rabbits in accordance with a 164 day immunization regimen, after which the animals that had produced specific antibodies were bled.

In order to sample the serum prior to immunization, 10 ml of blood per rabbit was taken as a preimmune control. Primary immunizations were carried out with Freund's complete adjuvant and subsequent boosts with incomplete Freund's adjuvant (IFA) (1 ml per rabbit, 0.5 ml per thigh muscle). Each injection consisted of approximately 200 µg of the purified peptide. At days 21, 42, and 70, a booster injection was given with IFA. At days 31, 42 and'80, 10 ml of blood was collected from the central ear artery for titer determination (6 ml/kg/rabbit). At day 80, the titer of the sera was checked, and 3 more injections were given (IFA) at 4 week intervals, followed by blood sampling 10 days later. 10 days after the last boost, anesthetized rabbits were exsanguinated via cardiac puncture, and antisera was collected.

### Immunization of goats

Goat polyclonal antibodies were generated according to standard methods. Three goats were immunized as follows. On day 1, all the goats received a primary immunization of 1 mg of YYR-KLH conjugates in complete Freund's adjuvant. Boosts were done by injection of 1 mg YYR-KLH in incomplete Freund's adjuvant for two of the three goats, whereas the third goat received 1 mg YYR-8map conjugates in incomplete Freund's adjuvant. Serum samples from each of the three bleeds were tested for reactivity by ELISA against YYR-BSA conjugates. From the third set of bleeds, total IgG was purified by ammonium sulfate precipitation and YYR-reactive IgG was purified using a YYR affinity column. IgG fractions were tested for reactivity to PrP^{Sc} as described herein. The exact immunization schedule was as follows: Day 1, primary immunization; Day 21, first boost immunization; Day 30, first bleed; Day 46, second boost immunization; Day 53, second boost immunization; Day 60, second bleed; Day 76, third boost immunization; Day 83, third boost immunization; and Day 90, third bleed.

Alternatively, monoclonal antibodies may be prepared using the synthetic peptides described herein and standard hybridoma technology (see, e.g., Kohler et al., *Nature* 256, 1975; Kohler et al., *Eur. J. Immunol.* 6:511, 1976; Kohler et al., *Eur. J. Immunol.* 6:292; 1976; Hammerling et al., *In Monoclonal Antibodies and T Cell Hydridomas,* Elsevier, NY, 1981; Ausubel et al., 1999, *Current Protocols in Molecular Biology,* Wiley Interscience, New York,). Once produced, monoclonal antibodies are also tested for specific PrP recognition by immunoprecipitation and western blot analysis (e.g., by using the methods described in Ausubel et al., *supra*).

### Immunization of mice

The generation of monoclonal antibodies was carried out as follows. Mice were immunized with baculovirus supernatant containing mouse PrP-AP fusion protein in complete Freund's adjuvant, then boosted 2 weeks later with the same antigen in incomplete Freund's adjuvant. Two weeks after that immunization the mice were boosted with a mixture of PrP-AP supernatant plus 100 µg of KLH-CYYRRYYRYY and 100 µg of KLH-CKYEDRYYRE conjugates. Splenocytes from these mice were fused to the FO murine B cell line (ATCC CRL-1646) to generate specific hybridoma clones. Hybridoma supernantants were screened by ELISA. There were no reactive supernatants to PrP-AP or to the CKYEDRYYRE sequence, although there were clones reactive to YYR-8map conjugates.

### Purification of antibody

Total rabbit IgG was purified from serum using the Pharmacia protein A HiTrap column according to the manufacturer's recommendations. Briefly, a HiTrap column was equilibrated with 3 column volumes of start buffer (0.2M sodium phosphate buffer, pH 7.0). Serum was applied, using a syringe through a luer adaptor, onto the column. The column was subsequently washed with 5 ml of start buffer. Bound protein was eluted with 0.1M glycine, pH 3.0, and collected in eppendorf tubes containing 1M Tris pH 8.0 (50 µl/500 µl sample). Fractions were analyzed on SDS-PAGE.

Goat polyclonal antibodies were purified from serum samples as is described above.

Mouse monoclonal antibodies were produced as ascites, and purified using a protein A column kit (Pierce) according to the manufacturer's instructions. Briefly, a sample of ascites was diluted with binding buffer at a 1:1 final ratio. The sample was then added to the top of the column, which had been previously equilibrated with binding buffer, and allowed to flow through the matrix. The pass-through material was collected and the column washed with 5 volumes of binding buffer. Mild elution buffer was added to the column to release the bound IgG antibody from the matrix. Other antibody isotypes were collected by switching to the IgG elution buffer. All the antibodies were collected in 1 ml fractions, which were analyzed by BCA to determine total protein content and SDS-PAGE electrophoresis to establish the degree of antibody purity. The fraction containing the most yield of IgG was desalted by passing it through a D-salt column (Pierce). The antibody fraction was allocated and stored at -80°C in PBS.

Antibodies produced using the afore-mentioned procedures were subsequently tested for high-affinity binding as follows.

### Preparation of bovine brain homogenates

Two methods were used for the preparation of bovine brain homogenates. In one method (A), brain samples were homogenized in tissue homogenization buffer (10% sucrose, 20 mM HEPES pH 7.5, 2% Sarcosyl, and 5 mM EDTA) using a Polytron (OMNI GLH). Homogenates were used at a final concentration of 1% (w/v).

In a second method (B), brain homogenate was prepared as a 10% (w/v) solution. Bovine brain was disrupted in a Dounce homogenizer (with a Teflon pestle) in 2 volumes of cold lysis buffer (100 mM NaCl, 10 mM EDTA, 0.5% Nonidet P-40, 0.5% sodium deoxycholate in tris-HCl, pH 7.4). The sample was incubated on ice for 20 minutes before applying 15 additional strokes in the homogenizer. Cellular debris was removed by centrifugation at 3000 rpm for 15 minutes at 4°C. The protein content of the supernatant was subsequently quantitated.

### Preparation of hamster and mouse homogenates

Hamster or mouse brain tissue was added to enough homogenization buffer (PBS 0.5% NP40,0.5% deoxycholate) to result in an 10% (weight/volume) homogenate. The tissue was homogenized by being repeatedly passed through an 18 gauge needle and a 22 gauge needle. Cellular debris was removed by two sequential centrifugations at 500g for 20 minutes. The total protein in the supernatant was quantitated with the BCA kit (Pierce), and the concentration was adjusted using homogenization buffer to a final concentration of 5 mg/ml. Aliquots containing 200 µl each were prepared and stored at -80°C.

### Magnetic bead conjugation

Sixty to 150 µg of purified antibody or BSA were conjugated to approximately 6 x 10⁸ tosylactivated magnetic beads (Dynal) using a protocol supplied by the manufacturer. Briefly, 1 ml of homogeneous unconjugated bead suspension per antibody was washed twice and resuspended in PBS pH 7.4 containing the antibodies or the BSA. The mixture was incubated at 37°C for 20-24 hours on a rotor. The mixture was then washed twice for 5 minutes with rotation in PBS 0.1% BSA and incubated in blocking buffer (0.2 M Tris pH 8.5 0.1% BSA) for 4 hours at 37°C with rotation. Following another 5 minute wash with PBS 0.1% BSA, the antibody-bead conjugates were washed in PBS 0.1% BSA 1% Tween-20 for 10 minutes, washed again with PBS 0.1% BSA and stored at 4°C.

### Proteinase K digestion

Bovine brain homogenate was incubated with proteinase K solution (100 µg/ml) at 50°C for 30 minutes. The digestion was stopped with the protease inhibitor, PMSF (2 mM).

Mouse and hamster brain homogenates were digested with 45 µg/ml final concentration Proteinase K for 30 minutes at 37 °C. The reaction was stopped with the addition of 19 mM PMSF final concentration.

### Immunoprecipitation

Ten µl of brain extract was added to 950 µl of immunoprecipitation buffer (PBS 3% NP-40, 3% Tween-20) and incubated at 37°C for 30 or 60 minutes. For experiments evaluating the reactivity of PrP 27-30 with the bead conjugates, the incubation was preceded by addition of 50 µl of 1 mg/ml proteinase K. Samples not treated with proteinase K were still incubated at 37°C for the appropriate time period. After the incubation, 60 µl of an 100 mM PMSF solution were added to both sets of tubes. One hundred µl of resuspended bead conjugates were then added to the mixture, and incubated with rotation at room temperature for 2 hours. The beads were washed 3 times with washing buffer (PBS 2% NP-40 2% Tween-20) and resuspended by vortex after each wash. After the last wash, the beads were resuspended in 20 µl of 2X loading buffer (100 mM Tris pH 6.8, 4% SDS, 0.015% bromphenol blue, 20% glycerol) and heated at 95°C for 3 minutes.

### Western Blot

The PrP^{Sc} content of brain homogenates was determined by western blotting according to standard methods. Protein samples were mixed with 2x sample buffer at a ratio of 1:1 and boiled for 5 minutes at 100°C. SDS-PAGE analysis was performed according standard methods. Samples were applied to a pre-cast 15% acrylamide gels (Biorad) along with pre-stained molecular weight markers (Biorad). The gels were run at 100V until the bromophenol blue dye front reached the bottom of the gel. The separated protein was then transferred onto PVDF membranes at 100 V for 1hr. The membrane was blocked for 30 minutes in blocking buffer, after which it was washed three times with TBST. The membranes were then incubated with an antibody specific for denatured PrP for 2 hours at room temperature. The membranes were washed as described above before incubation with a goat anti-mouse IgG alkaline phosphatase conjugated secondary antibody (1:5000 in TBST) for 1 hour at room temperature. After washing, signals were developed with the chemiluminescent substrate CDP-star, and exposed to X-ray films.

### Flow cytometry

Spleen cell suspensions were prepared from Balb/c mice by passing the tissues through a wire mesh. The cells were washed once with cold Dulbecco's PBS without Ca²⁺ or Mg²⁺ and viable cells were isolated by underlayering of the cell suspension with Lympholyte (Cedarlane) and centrifugation at 1300g for 20 minutes. The cells were washed once with cold Dulbecco's PBS without Ca²⁺ or Mg²⁺ 2.5% fetal bovine serum, and 0.5 x 10⁶ cells were aliquoted per well in a round bottom 96 well plate. The cells were centrifuged and resuspended in 50 µl of antibody-FITC conjugates at 1/10 final concentration in Dulbecco's PBS without Ca²⁺ or Mg²⁺ 2.5% fetal bovine serum, for 15 minutes on ice. The cells were then washed twice with cold Dulbecco's PBS without Ca²⁺ or Mg²⁺ 2.5% fetal bovine serum and resuspended in the same medium containing 1 µg/ml of propidium iodide. The cells were analyzed on a Coulter Epics flow cytometer and were gated by size and granularity (forward and side scatter) and viability (exclusion of propidium iodide fluorescence).

### FTTC antibody conjugation

Fluoresceinated mAbs were made by using the Fluorotag kit (Sigma) following the manufacturer's instructions. Briefly, 0.5 mg of each antibody was raised to pH 9 with concentrated bicarbonate buffer, and FITC stock solution was added to produce an FITC: antibody ratio of 20:1. The vials were then incubated for 2 hours at room temperature. Labeled antibody was separated from free FITC by passing the mixture over a Sephadex G-25M column. Conjugated antibodies were tested for successful fluoresceination by measuring their FITC emissions at 535 nm using an LJL Biosystems Analyst, and the antibodies were tested for retention of their binding activity with an ELISA against YYR-8map conjugates.

### Determination of PrP content of the brain homogenates

The presence of both PrP^{C} and PrP^{Sc} in extracts prepared from brain samples was confirmed by Western blotting using mAb6H4 (Figure 3). The dispersed banding pattern of PrP^{C} (33-35 kDa) was observed in extracts prepared from both normal and BSE-infected brain samples. After treatment of the extracts with proteinase K, PrP^{C} was digested completely, whereas PrP^{Sc} appeared as a 27-30 kDa band.

All brains were characterized for the presence of PrP^{Sc}, which accounted for 15-20% of the total PrP content in the pooled extracts (Figure 15).

### Soluble protocadherin-2 (sPC2) expression

PC2, a PrP binding protein, was used as a capture reagent for PrP^{C} and PrP^{Sc} to demonstrate PrP^{Sc} specificity of pAbC2. A plasmid (HU-PC43 3'trunc/PClnel) containing the human protocadherin-2 sequence coding for all six cadherin domains, but truncated at the start of the transmembrane domain, was transfected into COS cells. The culture medium containing the soluble form of protocadherin-2 was collected, and the presence of sPC2 was determined by western blotting using an anti-PC2 monoclonal antibody. WO 97/45746, entitled, "Prion Protein Binding Proteins and Uses Thereof," describes the use of PC2 as a receptor for the prion protein, and is hereby incorporated by reference.

### Testing of pAbC2 in an ELISA

To determine whether pAbC2 was useful in specifically recognizing PrP^{Sc} from bovine brain extracts, compared to PrP^{C} using recombinant PrP (rbPrP), an ELISA approach was used. Either pools of PrP^{Sc}-containing brain extracts or rbPrP was used to test the specificity of pAbC2 for PrP^{Sc}.

The wells of an Immunolon ELISA plate (Dynex) were coated overnight at 4°C with the PC2-containing culture supernatant in a TBS buffer containing 50 mM Tris, pH 7.5, 150 mM NaCl, 1 mM CaCl₂. For BSE-brain extract experiments, control wells were coated with a supernatant containing Mek-4; for rbPrP experiments, milk was used as a control to determine the non-specific binding of the antibody to the well. The coating of the ELISA plates with soluble PC2 was confirmed with an anti-PC2 monoclonal antibody. The wells were washed four times using a SLT 'Columbus' microplate washer (Tecan) with TBS containing 0.05% Tween 20, and blocked by filling the wells with 0.2% 1-Block (Tropix) in TBST and incubating the plate at 37°C for 1 hour. The plates were washed and the bovine brain homogenate (diluted to 1% w/v in TBS) or rbPrP was added to designated wells and incubated at RT for 1h. Wells were washed four times with TBST. pAbC2 was added to appropriate wells and incubated at RT for 1 hour, followed by a further 45 minute incubation with 100 µl of an anti-rabbit or mouse IgG/horseradish peroxidase conjugate (1:5000) in TBST containing 1% nonfat milk. Wells were washed four times with TBST. Signals were developed with TMB/H₂O₂ as a substrate for peroxidase. Reactions were stopped after 15 minutes by the addition of 100 µl of 2 M phosphoric acid. Signals were monitored at 450 nm with reference at 620 nm using a SLT microplate reader. Specific positive signals were determined by comparing PrP binding to PC2 with PrP binding to the negative control, Mek-4 or milk. Preimmune controls showed no binding.

In all cases of BSE-infected bovine brain extracts, the recorded absolute values for these points were higher than the recordings for the extracts from normal brain, regardless of the presence of sPC2 on the plates. It is conceivable that aggregation in the BSE samples resulted in nonspecific adherence to wells of the ELISA plates, hence, higher signals were recorded. However, values for BSE samples nonetheless were greater than those obtained in the same samples probed with the Mek-4 control.

The pAbC2 reacted more strongly to PC2-bound material than to Mck4-bound material, often 1.5-2 times greater than binding to Mek-4 control, suggesting specific binding to PrP^{Sc} (Figure 16). pAbC2 antibody as a secondary detection reagent bound only to PrP^{Sc}-positive samples in most cases, suggesting that this combination of reagents may enable PrP^{Sc} detection in the absence of a protease pre-treatment.

### PrP detection assay using immunoprecipitation

Once it was established that pAbC2 recognized PrP^{Sc} in the ELISA assays, an immunoprecipitation was subsequently done to verify whether pAbC2 could immunoprecipitate PrP from bovine brain extracts. Pools of several normal or BSE-infected brain extracts were used in the experiments, unless indicated otherwise. All brain samples had previously been characterized for the presence of PrP^{Sc}. Briefly, 20 µl of a 10% brain homogenate was incubated for 2 h at RT with various antibodies that are known to bind to PrP, such as mAb6H4, as well as pAbC2 in TBS containing 0.5M GuHCl. After incubation with 25 µl of magnetic protein A- or protein G-coupled beads (Dyna-beads) for 1 hour at room temperature, agarose beads were pelleted using a magnet. Pellets were washed three times and then boiled in SDS-sample buffer for analysis on western blots. PrP was detected with an anti PrP polyclonal or monoclonal antibody.

Using pAbC2, PrP was not detected in reactions containing extracts from normal brain, however, bands migrating at a position similar to PrP were detected in reactions containing extracts from BSE-infected samples (Figure 7). These results further substantiated the ELISA studies showing that pAbC2 is specific for PrP^{Sc}, and not for PrP^{C}. Uncoupled beads were used as a negative control, and showed no precipitation of PrP^{Sc}. mAb 6H4 was used as a positive control in the immunoprecipitation reactions. As expected, PrP^{C} was efficiently precipitated from normal brain using mAb 6H4.

### Use

The PrP peptides and PrP^{Sc}-specific antibodies described herein may be used, for example, for the following diagnostic, therapeutic, vaccine, and decontamination purposes, as well as for screening for novel compounds that can be utilized to diagnose or combat prion diseases or decontaminate prion samples.

### Test kits for the diagnosis of prion diseases

Epitope-specific anti-PrP antibodies find diagnostic use generally in the detection or monitoring of prion diseases. For example, anti-PrP antibodies may be used to monitor the presence or absence of PrP^{Sc} in a biological sample (e.g., a tissue biopsy, a cell, or fluid) using standard detection assays. Immunological assays may involve direct detection of PrP^{Sc}, and are particularly suited for screening large amounts of samples for the presence of PrP^{Sc}. For example, polyclonal or monoclonal antibodies produced against a continuous YYX epitope such as YYR (as described above) may be used in any standard immunoassay format (e.g., ELISA, Western blot, immunoprecipitation, flow cytometry, or RIA assay) to measure complex formation. In addition, if desired, because of the specificity of the antibodies described herein for PrP^{Sc}, pretreatment of a test sample with protease prior to immunological analysis may be omitted. Any appropriate label which may be directly or indirectly visualized may be utilized in these detection assays including, without limitation, any radioactive, fluorescent, chromogenic (e.g., alkaline phosphatase or horseradish peroxidase), or chemiluminescent label, or a hapten (for example, digoxigenin or biotin) which may be visualized using a labeled, hapten-specific antibody or other binding partner (e.g., avidin). Exemplary immunoassays are described, e.g., in Ausubel et al., *supra,* Harlow and Lane, *Antibodies: A Laboratory Approach, Cold Spring Harbor Laboratory, New York* (1988), and Moynagh and Schimmel, *Nature* 400:105, 1999. For example, using the antibodies described herein, PrP^{Sc} is readily detected at the cell surface (e.g., a leukocyte) using standard flow cytometry methods such as those described herein. Samples found to contain increased levels of labeled complex compared to appropriate control samples are taken as indicating the presence of PrP^{Sc}, and are thus indicative of a prion-related disease.

In addition, novel compounds useful for diagnosing prion disease may be identified using the antibodies of the invention. For example, combinatorial chemical libraries or small molecule libraries are screened to identify compounds having the ability to inhibit the binding interaction of one or more anti-YYX antibodies to a YYX epitope according to standard methods (e.g., equilibrium dialysis, Biacore analysis, or competitive inhibition). Such libraries may be derived from natural products, synthetic (or semi-synthetic) extracts, or chemical libraries according to methods known in the art. Those skilled in the field of drug discovery and development will understand that the precise source of compounds is not critical to the screening procedure(s) of the invention. Examples of natural compound sources include, but are not limited to, plant, fungal, prokaryotic, or animal sources, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries may be obtained commercially or may be produced according to methods known in the art. Furthermore, if desired, any library or compound is readily modified using standard chemical, physical, or biochemical methods.

Compounds that inhibit binding of an anti-YYX antibody to an YYX epitope at lowest concentration are referred to as "high affinity competitors" and are useful in the diagnotic methods of the invention. Such high affinity competitors that mimic the activity of the anti-YYX antibody (for example, the complementarity determining region ("CDR") of an anti-YYX antibody) are subsequently tested for efficient recognition and binding of PrP^{Sc}. Once identified, high affinity competitors may be coupled to solid substrates (for example, ELISA wells or beads) for use in the capture phase of virtually any diagnostic test for prion infection.

### Conversion of anti-PrP^{Sc} monoclonal antibody to IgM

Diagnostic methods may also be carried out using IgM antibodies, which have increased avidity for PrP^{Sc}. In order to increase antibody avidity for PrP^{Sc}, a specific binding IgG Fab region gene may be constructed into a pentavalent IgM fragment as follows.

Immunoglobulin (Ig) molecules are composed of distinct structural and functional domains. Each immunoglobulin is made up of 2 heavy chains (50-80kD each) and 2 light chains (25kD each). The heavy chains are a product of the rearrangement of one of four types of immunoglobulin genes, the V_{H}, D_{H}, J_{H} and C_{H}. The first three genes, collectively known as the variable region, combine to create the binding site of the heavy chain. The fourth gene, C_{H}, also known as the constant region, is not involved in the generation of the binding site but contributes to the heavy chain's recognition by other components of the immune system. Similarly, the light chain is derived from the rearrangement of one copy of 3 types of immunoglobulin genes, the V_{L}, J_{L}, and C_{L}. As for the heavy chain, the binding site of the light chain is generated by the combination of the V_{L} and J_{L} genes, whereas the C_{L} gene is not involved. The binding site of any given antibody is thus produced by the interaction of two variable regions: the V_{H}-D_{H}-J_{H} polypeptide and the V_{L}-J_{L} polypeptide. Most antibodies have two such binding sites. - During an immune response the first type of immunoglobulin made is called IgM. Generally IgMs contain binding sites with relatively low affinities, but compensate for that characteristic by expressing five binding sites per antibody molecule. As the immune response progresses, other antibodies such as IgGs are generated, which contain much higher affinities than the IgMs that came before, but only two binding sites per molecule.

As noted above, the tripeptide YYR or related motifs appear three times in the PrP sequence. In bovine, rodent and human PrP the motif appears twice as YYR and once as YYX. It is possible, therefore, as more of these individual motifs are detected by a particular reagent, the more sensitive that reagent will be. The highest affinity binding sites are contained within IgG molecules. However, as they only have two binding sites, these antibodies may not be the optimum reagents for detection of PrP^{Sc}. IgM molecules contain enough binding sites, but they are of lower affinity, therefore they are not the optimum reagents for detection of PrP^{Sc}. Modular construction of an Ig molecule therefore provides a solution to this problem, and a way to construct an optimum Ig for the detection of PrP^{Sc}.

The YYR-KLH antigen that was used to generate the anti-PrP^{Sc} polyclonal antibody is used to immunize mice. The mice are immunized using protocols established in the field that are known to result in high affinity IgG antibodies. B cell hybridomas are generated by standard procedures, and tissue culture supernatants generated from these cells that contain their secreted monoclonal antibodies are tested for immunoreativity to the YYR moiety of the antigen by ELISA. The rearranged immunoglobulin genes that produce the antibody are cloned using a one-sided PCR protocol as described in Heinrichs et al. *(J. Immunol. Methods,* 178:241-51, 1995). Only the variable regions of the heavy and light chains need to be cloned. These are then inserted, using standard procedures, into expression vector plasmids containing an appropriate constant region, in this case, the secretory version of an IgM constant region, but could be any other Ig isotype. This region may be obtained either from mouse or human origin, the latter to humanize the antibody such that it results in minimal side effects after administration to humans (for reviews see Winter and Harris, *Immunol Today,* 14(6):243-6, 1993; Vaughan et al., *Nature Biotech,* 16(6):535-9, 1998). The vectors, which now contain variable regions derived from IgG molecules coupled to constant regions derived, e.g., from IgM molecules, are then used to drive the generation of recombinant anti-PrP^{Sc} antibodies either in bacterial or eukaryotic expression systems. For an example of such a strategy see Poul et al., *Immunotechnology,* 1:189-96, 1995.

Alternatively, the variable region combination yielding the best reactivity against the YYR moiety of the YYR-KLH antigen will be selected from a phage display library as described in the literature (Marks et al., *J. Mol. Biol,* 222:581-597, 1991; Vaughan et al., *Nature Biotech,* 14:309-14, 1996) before undergoing the isolation and subcloning into a full length antibody described above.

### Conversion of anti-PrP^{Sc} monoclonal antibody to IgE

An alternative method to detect PrP^{Sc} in a sample (e.g., a blood sample) involves the conversion of PrP^{Sc} monoclonal antibody to IgE. PrP^{Sc} forms aggregates of various sizes that normally are not made with PrP^{C}. These PrP^{Sc} multimers may exist in blood of infected individuals. This characteristic may be used to detect PrP^{Sc} with a bioassay. Specifically, monoclonal antibodies that are either specific for PrP^{Sc}, or that are cross reactive with PrP^{C} and PrP^{Sc} are converted, by subcloning, into the IgE isotype. This would involve the same methods as described above, using an IgE constant region instead of an IgM constant region.

IgE antibodies are bound by cell surface receptors specific to the IgE constant region. These receptors are widely distributed in the body and play a central role in allergic reactions. The receptor under consideration here, the high-affinity receptor for IgE (Fc RI), is found on mast cells, basophils, eosinophiles, monocytes and Langerhans cells. It is a cell surface receptor composed of 3 polypeptide chains, and displays an exquisite affinity for IgE (Kₐ = 10^{-10M}). Each Fc RI binds one molecule of IgE (Kulczycki and Metzger, J. Exp. Med., 140: 1676, 1974; Barclay et al., The Leukocyte Antigen Factsbook, San Diego: Academic Press, 1997). However, in order for a signaling response to be initiated, multiple Fc RI receptors are crosslinked by a multivalent antigen (Metzger, J. Immunol, 149: 1477, 1992). The intracellular signal intensity is proportional to the degree of cross linkage. Once this occurs, the cell expressing the Fc RI degranulates, causing a rapid release of histamines and other stored mediators.

In the present bioassay method, a blood sample is incubated with a monoclonal antibody reactive to PrP^{Sc}, or cross reactive with PrP^{C} and PrP^{Sc}. Monomeric PrP and polymeric PrP (e.g., PrP^{Sc} aggregates) is bound, and the mixture is then incubated with a cell line expressing Fc RI, such as RBL-2H3, known to express 2-3 x 10⁵ Fc RI per cell (Barsumian et al., Eur. J. Immunol, 11: 317, 1981). Such a cell line is available from ATCC. Since aggregation of the Fc RI is required for degranulation to occur, monomeric PrP, whether bound by the antibody or not, will not cause cellular degranulation, however; polymeric PrP will. The released mediators are detected directly in a standard immunological assay, e.g., by ELISA.

### PrP^{Sc} vaccines

Peptides described herein and mixtures and combinations thereof are also useful as active components of vaccines capable of inducing a prophylactic or therapeutic immune response against prion diseases in hosts susceptible to and/or harboring infection. Routes of administration, antigen doses, number and frequency of injections will vary from species to species and may parallel those currently being used in the clinic and/or experimentally to provide immunity or therapy against other infectious diseases or cancer. For example, the vaccines are pharmaceutically acceptable compositions containing the peptide of this invention, its analogues or mixtures or combinations thereof, in an amount effective in the mammal, including a human, treated with that composition to raise immunity sufficient to protect the treated mammal from prion infection for a period of time. It is also possible that PrP^{Sc}-specific immunity prompted by immunization with YYX (YYR, or YYD, or YYQ) or related compounds are useful to favor the degradation of PrP^{Sc} or alleviate manifestations of the disease without affecting the expression or function of PrP^{C} in the brain and other tissues, resulting in improvement of clinical status in clinically symptomatic humans with prion disease.

Different types of vaccines can be developed according to standard procedures known in the art. For example, a vaccine may be peptide-based, nucleic acid-based, bacterial- or viral-based vaccines. More specifically, with regard to peptide vaccines, peptides corresponding to the PrP^{Sc}-specific epitope or a functional derivatives thereof can be utilized as a prophylactic or therapeutic vaccine in a number of ways, including: 1) as monomers or multimers of the same sequence, 2) combined contiguously or non-contiguously with additional sequences that may facilitate aggregation, promote presentation or processing of the epitope(e.g., class I/II targeting sequences) and/or additional antibody, T helper or CTL epitopes to increase the immunogenicity of the PrP^{Sc} -specific epitope as a means to enhance efficacy of the vaccine, 3) chemically modified or conjugated to agents that would increase the immunogenicity or delivery of the vaccine (e.g., fatty acid or acyl chains, KLH, tetanus toxoid, cholera toxin, etc.), 4) any combination of the above, 5) the above in combination with adjuvants, including but not limited to aluminum salts, saponins or triterpenes, MPL, and cholera toxin, and/or delivery vehicles, including but not limited to liposomes, VPLs or virus-like particles, microemulsions, attenuated or killed bacterial and viral vectors, and degradable microspheres, 6) administered by any route or as a means to load cells with antigen *ex vivo.*

Examples of uses of nucleic-acid based vaccines as a prophylactic or a therapeutic include: 1) any nucleic acid encoding the expression (transcription and/or translation) of the PrP^{Sc}-specific epitope, 2) additional nucleic acid sequences that facilitate processing and presentation, aggregation, secretion, targeting (to a particular cell type) of the PrP^{Sc}-specific epitope, either translational fusions or independent transcriptional units, 3) additional nucleic acid sequences that function as adjuvants/immunomodulators, either translational fusions or independent transcriptional units, 4) additional antibody, T helper or CTL epitopes that increase the immunogenicity of the PrP^{Sc}-specific epitope or efficacy of the vaccine, either translational fusions or independent, 5) any combination of the above, 6) the above administered in saline ('naked' DNA) or in combination with an adjuvant(s), (e.g. aluminum salts, QS-21, MPL), immunomodulatory agent(s) (e.g. rIL-2, rGM-CSF, rIL-12), and/or nucleic acid delivery agents (e.g. polymer-, lipid-, peptide-based, degradable particles, microemulsions, VPLs, attenuated bacterial or viral vectors) using any route or *ex vivo* loading.

Attenuated or killed bacterial or viral vectors can be used to deliver either the antigen or DNA/RNA that codes for the expression of the antigen. These can also be used as a means to load cells with antigen *ex vivo.*

Vaccines are prepared according to standard methods known in the art, and will be readily applicable to any new or improved method for vaccine production.

### Prion Decontamination

The methods and compositions described herein are useful for the decontamination of biological samples that are known or suspected of being contaminated with a prion, e.g. intended for transplantation. In particular, biological samples may be incubated with anti-PrP^{Sc} antibody, and the complexes removed using standard methods. Alternatively, anti-PrP^{Sc} antibodies may be incubated with biological samples to complex with, and thereby inhibit the infectivity of prion.

### Prion Disease Therapeutics

The methods and compositions of the invention also provide a means for treating or preventing prion diseases in mammals including, without limitation, humans, sheep, pigs, cattle, goats, dogs, cats, and pet species. As noted above, it is possible that changes in the orientation of tyrosine side chains in the tyrosine dimers, or clustering of YYX epitopes (e.g., YYR/D/Q) might contribute to the change in physicochemical properties of PrP upon conversion to PrP^{Sc}, such as hydrophobicity and tendency to aggregate. It is also possible that these residues might be critical in the PrP^{C} to PrP^{Sc} conversion reaction. If this can be shown, then treatments for prion diseases can be based upon antagonists that disrupt, suppress, attenuate, or neutralize the biological events associated with PrP^{C} to PrP^{Sc} conversion. Antibodies actively produced from YYX (e.g., YYR) peptide immunization, or passive transfer of polyclonal or monoclonal antibodies against YYX, are useful in treating these diseases. Moreover, the invention includes not only an intact monoclonal antibody, but also an immunologically active antibody fragment. Examples of such a fragment include a Fab or (Fab)₂ fragment, an engineered single chain Fᵥ molecule, and a chimeric antibody (such as a "humanized" antibody). The term "humanized antibody," as used herein, refers to antibody molecules in which the amino acid sequence in the non-antigen binding regions has been altered so that the antibody more closely resembles a human antibody, and still retains its original binding ability. Humanized forms of non-human (e.g., murine) antibodies are constructed and characterized according to standard methods known in the art, for example, those described in Kutemeier et al. (*Biotechniques* 17:242-246, 1994); Major et al.(*Hum. Antibodies Hybridomas* 5:9-17, 1994); Jolliffe (*Int. Rev. Immunol.* 10:241-250, 1993); Carter et al. (*Biotechnology* 10:163-167, 1992); Miyachi et al. (*J. Clin. Lab. Anal.* 6:343-50); and Leung et al. (*Mol. Immunol.* 32:1413-1427, 1995). Humanized antibodies are less likely to be immunogenic and are useful in passive immunotherapies. Furthermore, a chimeric antibody of the invention may, if desired, include a variable region of a non-human antibody, e.g., a murine variable region, and a constant region of a human antibody. In some embodiments of the invention, an antibody or antibody fragment is linked to a detectable label. Examples of detectable labels include a radioactive label, a non-radioactive isotopic label, a fluorescent label, an enzyme label, and a colorimetric label.

Moreover, small molecules derived from the structure of the YYR epitope(s), including but not limited to tyrosine side-chain derivatives, may block the conversion reaction. Finally, direct chemical modification of critical residues, such as enzymatic lysis of tyrosine rings, or covalent derivatization of tyrosine rings with bulky substitutions, may also disrupt the PrP^{C} to PrP^{Sc} conversion reaction if amino acids in the YYR epitope(s) prove to be critical in the conversion process.

For example, such compounds may be identified using the antibodies of the invention. Accordingly, combinatorial libraries or small molecule libraries or both (*infra*) are screened to identify compounds having the ability to inhibit the binding interaction one or more anti-YYX antibodies to a YYX epitope according to standard methods (e.g. equilibrium dialysis, Biacore analysis, or competitive inhibition). Compounds that inhibit binding of such an antibody are useful in the therapeutic methods of the invention. Once identified, such compounds are tested for their ability to combat prion diseases in any appropriate model system.

Evaluation of whether a test antagonist confers protection against the development of a prion disease *in vivo* generally involves using an animal known to develop such a disease (e.g., Chandler, *Lancet* 6:1378-1379, 1961; Eklund et al., *J. Infectious Disease* 117:15-22, 1967; Field, *Brit. J. Exp. Path.* 8:129-239, 1969). An appropriate animal (for example; a mouse or hamster) is treated with the test compound according to standard methods, and a reduced incidence or delayed onset or progression of a prion-related illness, compared to untreated control animals, is detected as an indication of protection. The test compound may be administered to an animal which has previously been injected with a prion agent or, alternatively, the test compound may be tested for its ability to neutralize a prion agent by pre-incubating the prion and the compound and injecting the prion/compound mixture into the test animal. A molecule (e.g., an antagonist as described above) that is used to treat or prevent a prion disease is referred to as an "anti-prion therapeutic."

Alternatively, it is possible that circulating antibodies reactive against PrP^{Sc} may act to accelerate the disease by stabilizing the conformation of PrP^{Sc}. Therefore, blocking the action of these endogenous antibodies may slow the disease progression or have other beneficial effects. The YYR-specific monoclonal antibodies may be used as substrates to raise another set of monoclonal antibodies reactive to the binding site of the YYR-specific antibodies. These second set of antibodies are known as anti-idiotypic. The anti-idiotype antibodies are useful to neutralize the circulating PrP^{Sc} reactive antibodies.

An anti-prion therapeutic according to the invention may be administered with a pharmaceutically-acceptable diluent, carrier, or excipient, in unit dosage form. For example, conventional pharmaceutical practice may be employed to provide suitable formulations or compositions to administer such anti-prion therapeutics to animals suffering from or presymptomatic for a prion disease, or at risk for developing a prion disease. Any appropriate route of administration can be employed, for example, parenteral, intravenous, subcutaneous, intramuscular, intracranial, intraorbital, ophthalmic, intraventricular, intracapsular, intraspinal, intracisternal, intraperitoneal, intranasal, aerosol, or oral administration.

Methods well known in the art for making formulations are found in, for example, "Remington's Pharmaceutical Sciences." Formulations for parenteral administration can, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers can be used to control the release of the compounds. Other potentially useful parenteral delivery systems for anti-prion therapeutic compounds include ethylene-vinyl acetate copolymer particles, osmotic pumps, implantable infusion systems, and liposomes. Formulations for inhalation can contain excipients, for example, lactose, or can be aqueous solutions containing, for example, polyoxyethylene-9-lauryl ether, glycocholate and deoxycholate, or can be oily solutions for administration in the form of nasal drops, or as a gel.

The methods of the present invention may be used to reduce or prevent the disorders described herein in any animal, for example, humans, domestic pets, or livestock. Where a non-human animal is treated, the anti-prion therapeutic employed is preferably specific for that species.

Other embodiments are within the scope of the following claims.

## Claims

1. Antibody or fragment thereof that binds with high binding affinity to a YYX epitope of a mammalian PrP^{Sc}.

2. The antibody of claim 1, wherein said antibody is a polyclonal antibody generated against a YYR epitope of PrP^{Sc}.

3. The antibody, of claim 2, wherein YYX epitope is part of CYYR (SEQ ID NO: 32).

4. The antibody of claim 1, wherein said antibody is a monoclonal antibody generated against a YYR epitope of PrP^{Sc}.

5. The antibody of claim 1, wherein said antibody is an IgG, IgM, IgE, IgD, or IgA.

6. The antibody of claim 1, wherein said antibody fragment is a Fab or Fv fragment.

7. A hybridoma cell line that produces a monoclonal antibody that binds with high binding affinity to a YYX epitope of a mammalian PrP^{Sc}.

8. The antibody of claim 1 or the hybridoma cell line of claim 7, wherein said antibody binds to a YYR epitope of a mammalian PrP^{Sc}.

9. The antibody of claim 4 or the hybridoma cell line of claim 7, wherein said YYR epitope is part of CYYRRYYRYY (SEQ ID NO: 33).

10. A composition comprising the antibody of any one of claims 1 to 6, 8, 9 or 20.

11. The composition of claim 10, wherein said composition further comprises a carrier.

12. The composition of claim 10, wherein said composition is a therapeutic composition.

13. An immunological test kit comprising the antibody of any one of claims 1 to 6, 8, 9 or 20 and a means for detecting said antibody.

14. A method for detecting PrP^{Sc} in a biological sample, said method comprising the steps of:
(a) contacting said biological sample with the antibody of claims 1 to 6, 8, 9 or 20 under conditions that allow for complex formation between said antibody and PrP^{Sc}; and
(b) detecting said complexes as an indication that PrP^{Sc} is present in said biological sample.

15. The method of claim 14, wherein said biological sample comprises a tissue or cell, a tissue or cell extract, a bodily fluid, or a biopsy.

16. The method of claim 14, wherein said PrP^{Sc} is from a human, a livestock species, or a pet species.

17. The method of claim 14, wherein said complex is detected using an ELISA, RIA, western blotting, immunopreciptation, or flow cytometry.

18. The antibody of claims 1 to 6, 8, 9 or 20 for use in treating or preventing a PrP^{Sc} disease in a mammal.

19. A method for generating an antibody that binds with high binding affinity to a mammalian PrP^{Sc}, said method comprising the steps of:
(a) providing a prion protein peptide comprising an accessible epitope having two or more amino acid side chains;
(b) immunising a non-human mammal with said prion protein peptide of step (a); and
(c) purifying an antibody that binds with high binding affinity to a YYX epitope of a mammalian PrP^{Sc} from a tissue of said mammal or from a hybridoma made using said tissue.

20. The antibody of claim 1, hybridoma of claim 7 or the methods of claim 14 or claim 19, wherein said antibody does not substantially bind PrP^{c}.

21. The method of claim 14 or claim 19, wherein said antibody is a polyclonal antibody or fragment thereof.

22. The method of claim 14 or claim 19, wherein said antibody is a monoclonal antibody or fragment thereof.

23. The method of claim 19, wherein said prion protein peptide comprises a YYX amino acid sequence.

24. The method of claim 23, wherein said prion protein peptide comprises a YYR or YYQ or YYD amino acid sequence.

25. The method of claim 24, wherein said peptide is composed of 18, 12, 8, 5 or fewer amino acids.

26. The method of claim 24, wherein said prion protein peptide consists of a YYR amino acid sequence.

27. The method of claim 19, wherein said prion protein peptide comprises a synthetic peptide having the formula:
A-Tyr-Tyr-B-(Tyr-Tyr-B)ₙ
wherein A is either any amino acid or is absent;
wherein B is either any amino acid or is absent; and
wherein n is from 0 to 10, inclusive; or a synthetic peptide of the formula:
A-Tyr-Tyr-B-C-Tyr-Tyr-D-Tyr-Tyr-(Tyr-Tyr-B)ₙ
wherein A is either any amino acid or is absent;
wherein B is either any amino acid or is absent;
wherein C is either any amino acid or is absent;
wherein D is either any amino acid or is absent; and
wherein n is 0 to 10, inclusive.

28. The method of claim 19, wherein said prion protein peptide comprises any one of the sequences set forth in SEQ ID NOs: 31-33 linked to an immunological carrier.

29. The method of claim 28, wherein said prion protein peptide consists of any one of the sequences set forth in SEQ ID NOs: 31-33 linked to an immunological carrier.

30. A method for decontaminating PrP^{Sc} from a biological sample, said method comprising the steps of:
(a) treating the biological sample with an antibody of any one of claims 1-6, 8-9 and 20 for a period of time sufficient to permit the formation of an anti-PrP^{Sc} antibody:PrP^{Sc} complex; and
(b) recovering said anti-PrP^{Sc} antibody:PrP^{Sc} complex from said biological sample.

31. A method of inhibiting PrP^{Sc} in a biological sample isolated from the human or animal body, said method comprising:
treating the biological sample with an antibody of any one of claims 1-6, 8-9 and 20 for a period of time sufficient to permit the formation of an anti-PrP^{Sc} antibody:PrP^{Sc} complex.

32. The method of claim 30 or claim 31, wherein said biological sample is a bodily fluid, a tissue or organ.

33. The method of claim 30 or claim 31, wherein said biological sample is perfused with said antibody.

34. Use of an antibody of any one of claims 1-6, 8-9 and 20 in the manufacture of a medicament for treating or preventing prion diseases.

35. The method of claim 26, wherein said prion protein peptide is linked to an immunological carrier.

## Patentansprüche

1. Antikörper oder ein Fragment davon, der mit hoher Bindungsaffinität an ein YYX Epitop eines Säugetier PrP^{Sc} bindet.

2. Antikörper nach Anspruch 1, wobei der genannte Antikörper ein gegen ein YYR Epitop von PrP^{Sc} erzeugter polyklonaler Antikörper ist.

3. Antikörper nach Anspruch 2, wobei YYX Epitop ein Teil von CYYR (SEQ ID No: 32) ist

4. Antikörper nach Anspruch 1, wobei der genannte Antikörper ein gegen ein YYR Epitop von PrP^{Sc} erzeugter monoklonaler Antikörper ist.

5. Antikörper nach Anspruch 1, wobei der genannte Antikörper ein IgG, IgM, IgE, IgD oder IgA ist.

6. Antikörper nach Anspruch 1, wobei das genannte Antikörperfragment ein Fab- oder Fv-Fragment ist.

7. Eine Hybridomzelllinie, welche einen monoklonalen Antikörper erzeugt, der mit hoher Bindungsaffinität an ein YYX Epitop eines Säugetier PrP^{Sc} bindet.

8. Antikörper nach Anspruch 1 oder die Hybridomzelllinie nach Anspruch 7, wobei der genannte Antikörper an ein YYR Epitop eines Säugetier PrP^{Sc} bindet.

9. Antikörper nach Anspruch 4 oder die Hybridomzelllinie nach Anspruch 7, wobei das genannte YYR Epitop ein Teil von CYYRRYYRYY (SEQ ID No: 33) ist.

10. Zusammensetzung, welche den Antikörper nach einem der Ansprüche 1 bis 6, 8, 9 oder 20 enthält.

11. Zusammensetzung nach Anspruch 10, wobei die genannte Zusammensetzung weiters einen Träger enthält.

12. Zusammensetzung nach Anspruch 10, wobei die genannte Zusammensetzung eine therapeutische Zusammensetzung ist.

13. Immunologische Testausrüstung, welche einen Antikörper nach einem der Ansprüche 1 bis 6, 8, 9 oder 20 und Mittel zur Auffindung des genannten Antikörpers enthält.

14. Verfahren zur Auffindung von PrP^{Sc} in einer biologischen Probe, wobei das genannte Verfahren die folgenden Schritte aufweist:
(a) In Kontakt Bringen der biologischen Probe mit dem Antikörper der Ansprüche 1 bis 6, 8, 9 oder 20 unter Bedingungen die eine Komplexbildung zwischen dem genannten Antikörper und PrP^{Sc} zulassen; und
(b) Ermittteln des genannten Komplexes als ein Hinweis darauf, dass PrP^{Sc} in der genannten biologischen Probe vorhanden ist.

15. Verfahren nach Anspruch 14, wobei die genannte biologische Probe ein Gewebe oder eine Zelle, ein Gewebe- oder Zellextrakt, eine Körperflüssigkeit oder eine Biopsie ist.

16. Verfahren nach Anspruch 14, wobei die genannte PrP^{Sc} von einem Menschen, einer Viehart oder einer Haustierart stammt.

17. Verfahren nach Anspruch 14, wobei der genannte Komplex unter Verwendung eines ELISA, RIA, Western Blotting, Immunfällung oder einer Flusscytometrie ermittelt wird.

18. Antikörper der Ansprüche 1 bis 6, 8, 9 oder 20 zur Verwendung bei der Behandlung oder Vorbeugung einer PrP^{Sc} Krankheit in einem Säugetier.

19. Verfahren zur Erzeugung eines Antikörpers, , der mit hoher Bindungsaffinität an Säugetier PrP^{Sc} bindet, welches Verfahren die folgenden Schritte aufweist:
(a) Bereitstellen eines Prionenproteinpeptids, welches ein zugängliches Epitop mit zwei oder mehr Aminosäurenseitenketten aufweist;
(b) Immunisieren eines nicht-menschlichen Säugetieres mit dem genannten Prionenproteinpeptid aus Schritt (a); und
(c) Reinigen eines Antikörpers, der mit hoher Bindungsaffinität an ein YYX Epitop eines Säugetier PrP^{Sc} bindet, aus einem Gewebe des Säugetieres oder einem Hybridom, das unter Verwendung des genannten Gewebes hergestellt wurde.

20. Antikörper nach Anspruch 1, Hybridom nach Anspruch 7 oder Verfahren nach Anspruch 14 oder Anspruch 19, wobei der genannte Antikörper nicht wesentlich an PrP^{c} bindet.

21. Verfahren nach Anspruch 14 oder Anspruch 19 , wobei der genannte Antikörper ein polyklonaler Antikörper oder ein Fragment davon ist.

22. Verfahren nach Anspruch 14 oder Anspruch 19 , wobei der genannte Antikörper ein monoklonaler Antikörper oder ein Fragment davon ist.

23. Verfahren nach Anspruch 19, wobei das genannte Prionenproteinpeptid eine YYX Aminosäuresequenz aufweist.

24. Verfahren nach Anspruch 23, wobei das genannte Prionenproteinpeptid eine YYR oder YYQ oder YYD Aminosäuresequenz aufweist.

25. Verfahren nach Anspruch 24, wobei das genannte Peptid aus 18, 12, 8, 5 oder weniger Aminosäuren zusammengesetzt ist.

26. Verfahren nach Anspruch 24, wobei das genannte Prionenproteinpeptid aus einer YYR Aminosäuresequenz besteht.

27. Verfahren nach Anspruch 19, wobei das genannte Prionenproteinpeptid ein synthetisches Peptid mit der Formel
A-Tyr-Tyr-B-(Tyr-Tyr-B)ₙ
aufweist,
worin A entweder irgendeine Aminosäure oder abwesend ist,
worin B entweder irgendeine Aminosäure oder abwesend ist, und
worin n von 0 bis einschließlich 10 beträgt; oder ein synthetisches Peptid der Formel
A-Tyr-Tyr-B-C-Tyr-Tyr-D-Tyr-Tyr-(Tyr-Tyr-B)ₙ
ist,
worin A entweder irgendeine Aminosäure oder abwesend ist,
worin B entweder irgendeine Aminosäure oder abwesend ist,
worin C entweder irgendeine Aminosäure oder abwesend ist,
worin D entweder irgendeine Aminosäure oder abwesend ist, und
worin n von 0 bis einschließlich 10 ist.

28. Verfahren nach Anspruch 19, wobei das genannte Prionenproteinpeptid irgendeine der in den SEQ ID No: 31-33 wiedergegebenen Sequenzen aufweist und an einen immunologischen Träger gebunden ist.

29. Verfahren nach Anspruch 28, wobei das genannte Prionenproteinpeptid aus irgendeine der in den SEQ ID No: 31-33 wiedergegebenen Sequenzen besteht und an einen immunologischen Träger gebunden ist.

30. Verfahren zum Entfernen von PrP^{Sc} aus einer biologischen Probe, wobei das Verfahren die folgenden Schritte aufweist:
(a) Behandeln der biologischen Probe mit einem Antikörper nach einem der Ansprüche 1-6, 8-9 und 20 für einen Zeitraum der ausreicht, die Bildung eines anti-PrP^{Sc}-Antikörper:PrP^{Sc}-Komplexes zuzulassen; und
(b) Gewinnen des genannten anti-PrP^{Sc}-Antikörper:PrP^{Sc}-Komplexes aus der biologischen Probe.

31. Verfahren zur Inhibierung von PrP^{Sc} in einer biologischen Probe, die aus einem menschlichen oder tierischen Körper isoliert ist, wobei das Verfahren aufweist:
Behandeln der biologischen Probe mit einem Antikörper nach einem der Ansprüche 1-6, 8-9 und 20 für einen Zeitraum der ausreicht, die Bildung eines anti-PrP^{Sc}-Antikörper:PrP^{Sc}-Komplexes zuzulassen.

32. Verfahren nach Anspruch 30 oder Anspruch 31, wobei die genannte biologische Probe eine Körperflüssigkeit, ein Gewebe oder ein Organ ist.

33. Verfahren nach Anspruch 30 oder Anspruch 31, wobei die genannte biologische Probe mit dem genannten Antikörper durchschwemmt wird.

34. Verwendung eines Antikörpers nach einem der Ansprüche 1-6, 8-9 und 20 bei der Herstellung eine Medikaments zur Behandlung oder Vorbeugung einer Prionen-Erkrankung.

35. Verfahren nach Anspruch 26, wobei das genannte Prionenproteinpeptid an einen immunologischen Träger gebunden wird.

## Revendications

1. Anticorps ou fragment d'anticorps qui se lie avec une haute affinité de liaison à un épitope YYX d'un PrP^{Se} de mammifère.

2. Anticorps selon la revendication 1, dans lequel ledit anticorps est un anticorps polyclonal généré contre un épitope YYR de prP^{sc}.

3. Anticorps selon la revendication 2, dans lequel l'épitope YYX fait partie de CYYR (SEQ ID NO:32).

4. Anticorps selon la revendication 1, dans lequel ledit anticorps est un anticorps monoclonal généré contre un épitope YYR de PrP^{sc}.

5. Anticorps selon la revendication 1, dans lequel ledit anticorps est une IgG, une IgM, une IgE, une IgD ou une IgA.

6. Anticorps selon la revendication 1, dans lequel ledit fragment d'anticorps est un fragment Fab ou Fv.

7. Lignée cellulaire d'hybridome qui produit un anticorps monoclonal qui se lie avec une haute affinité de liaison à un épitope YYX d'un PrP^{sc} de mammifère.

8. Anticorps selon la revendication 1 ou lignée cellulaire d'hybridome selon la revendication 7, dans lequel ledit anticorps se lie à un épitope YYR d'un PrP^{sc} de mammifère.

9. Anticorps selon la revendication 4 ou lignée cellulaire d'hybridome selon la revendication 7, dans lequel ledit épitope YYR fait partie de CYYRRYYRYY (SEQ ID NO:33).

10. Composition comprenant l'anticorps selon l'une quelconque des revendications 1 à 6, 8, 9 ou 20.

11. Composition selon la revendication 10, dans laquelle ladite composition comprend en outre un porteur.

12. Composition selon la revendication 10, dans laquelle ladite composition est une composition thérapeutique.

13. Trousse de test immunologique comprenant l'anticorps selon l'une quelconque des revendications 1 à 6, 8, 9 ou 20 et un moyen pour détecter ledit anticorps.

14. Procédé de détection de PrP^{sc} dans un échantillon biologique, ledit procédé comprenant les étapes de :
(a) mise en contact dudit échantillon biologique avec l'anticorps selon les revendications 1 à 6, 8, 9 ou 20, dans des conditions qui permettent la formation de complexe entre ledit anticorps et PrP^{sc} ; et
(b) détection desdits complexes comme indication de la présence de PrP^{Sc} dans ledit échantillon biologique.

15. Procédé selon la revendication 14, dans lequel ledit échantillon biologique comprend un tissu ou une cellule, un extrait tissulaire ou cellulaire, un fluide corporel ou une biopsie.

16. Procédé selon la revendication 14, dans lequel PrP^{sc} provient d'un humain, d'une espèce de bétail ou d'une espèce d'animal domestique.

17. Procédé selon la revendication 14, dans lequel ledit complexe est détecté par ELISA, RIA, buvardage Western, immunoprécipitation ou cytométrie de flux.

18. Anticorps selon les revendications 1 à 6, 8, 9 ou 20, pour usage dans le traitement ou la prévention d'une maladie à PrP^{sc} chez un mammifère.

19. Procédé pour générer un anticorps qui se lie avec une haute affinité de liaison à un PrP^{Sc} de mammifère, ledit procédé comprenant les étapes de :
(a) fourniture d'un peptide de prion comprenant un épitope accessible ayant deux chaînes latérales d'acides aminés ou plus;
(b) immunisation d'un mammifère non humain avec ledit peptide de prion de l'étape (a); et
(c) purification d'un anticorps qui se lie avec une haute affinité de liaison à un épitope YYX d'un PrP^{sc} de mammifère d'un tissu dudit mammifère ou d'un hybridome fait en utilisant ledit tissu.

20. Anticorps selon la revendication 1, hybridome selon la revendication 7 ou procédés selon la revendication 14 ou la revendication 19, dans lesquels ledit anticorps ne se lie pas substantiellement à PrP^{Sc}.

21. Procédé selon la revendication 14 ou la revendication 19, dans lequel ledit anticorps est un anticorps polyclonal ou un fragment de celui-ci.

22. Procédé selon la revendication 14 ou la revendication 19, dans lequel ledit anticorps est un anticorps monoclonal ou un fragment de celui-ci.

23. Procédé selon la revendication 19, dans lequel ledit peptide de prion comprend une séquence d'acides aminés YYX.

24. Procédé selon la revendication 23, dans lequel ledit peptide de prion comprend une séquence d'acides aminés YYR ou YYQ ou YYD.

25. Procédé selon la revendication 24, dans lequel ledit peptide est composé de 18, 12, 8, 5 acides aminés ou moins.

26. Procédé selon la revendication 24, dans lequel ledit peptide de prion est constitué d'une séquence d'acides aminés YYR.

27. Procédé selon la revendication 19, dans lequel ledit peptide de prion comprend un peptide de synthèse ayant la formule :
A-Tyr-Tyr-B-(Tyr-Tyr-B)ₙ
dans laquelle
A est un acide aminé quelconque ou est absent;
B est un acide aminé quelconque ou est absent; et
n est de 0 à 10, inclus; ou un peptide de synthèse de formule :
A-Tyr-Tyr-B-C-Tyr-Tyr-D-Tyr-Tyr-(Tyr-Tyr-B)ₙ
dans laquelle
A est un acide aminé quelconque ou est absent;
B est un acide aminé quelconque ou est absent;
C est un acide aminé quelconque ou est absent;
D est un acide aminé quelconque ou est absent; et
n est de 0 à 10, inclus.

28. Procédé selon la revendication 19, dans lequel ledit peptide de prion comprend l'une quelconque des séquences représentées dans les SEQ ID NO:31-33 liée à un porteur immunologique.

29. Procédé selon la revendication 28, dans lequel ledit peptide de prion est constitué de l'une quelconque des séquences représentées dans les SEQ ID NO:31-33 liée à un porteur immunologique.

30. Procédé pour décontaminer PrP^{Sc} dans un échantillon biologique, ledit procédé comprenant les étapes de :
(a) traitement de l'échantillon biologique par un anticorps selon l'une quelconque des revendications 1 à 6, 8, 9 ou 20 pendant une période suffisante pour permettre la formation d'un complexe anticorps anti-PrP^{sc}/PrP^{sc} ; et
(b) récupération dudit complexe anticorps anti-PrP^{sc}/PrP^{sc} dans ledit échantillon biologique.

31. Procédé d'inhibition de PrP^{sc} dans un échantillon biologique isolé du corps d'un humain ou d'un animal, ledit procédé comprenant :
le traitement de l'échantillon biologique avec un anticorps selon l'une quelconque des revendications 1 à 6, 8, 9 ou 20, pendant une période de temps suffisante pour permettre la formation d'un complexe anticorps anti-PrP^{sc}/PrP^{sc}.

32. Procédé selon la revendication 30 ou la revendication 31, dans lequel ledit échantillon biologique est un fluide corporel, un tissu ou un organe.

33. Procédé selon la revendication 30 ou la revendication 31, dans lequel ledit échantillon biologique est perfusé avec ledit anticorps.

34. Utilisation d'un anticorps selon l'une quelconque des revendications 1 à 6, 8, 9 ou 20 dans la fabrication d'un médicament pour le traitement ou la prévention des maladies à prion.

35. Procédé selon la revendication 26, dans lequel ledit peptide de prion est lié à un porteur immunologique.
